# EUROPEAN PATENT APPLICATION

(11) **EP 4 173 605 A1**
(43) Date of publication of application: **03.05.2023**
(21) Application number: 21875815.9
(22) Date of filing: 30.09.2021
(51) Int. Cl.: A61F 13/15

(54) **ABSORBENT ARTICLE AND ABSORBENT ARTICLE PACKAGE**

(30) Priority: 30.09.2020 CN 202011057366
(71) Applicant: Unicharm Corporation, Ehime 799-0111 (JP)
(72) Inventor: KAWAKAMI, Yusuke, Kanonji-shi, Kagawa 769-1602 (JP); WANG, Yinhua, Shanghai 201700 (CN); ZHENG, Lingshuang, Shanghai 201700 (CN)
(74) Representative: Dolleymores
(86) International application number: PCT/JP2021/036273
(87) International publication number: WO 2022/071519

(57) **Abstract**

An absorbent article (1) that comprises an absorbent body (10) and exterior members (30, 40) provided on the non-skin side of the absorbent body (10), wherein the exterior members (30, 40) include hydrophilic non-woven fabric parts (32, 42) on at least a part of the most non-skin side surface. The hydrophilic non-woven fabric parts (32, 42) are disposed so that a value, which is calculated by dividing the difference between the weight of the absorbent article (1) when the packaged absorbent article (1) is opened and the weight of the absorbent article (1) in a dried state by the weight of the absorbent article (1) in the dried state, is 0.1 or less.

## Description

### FIELD

The present invention relates to an absorbent article and an absorbent-article packaged piece.

### BACKGROUND

There has been conventionally known an underpants-shaped diaper using a hydrophilic nonwoven fabric having enhance
d hydrophilicity. For example, Patent Literature 1 discloses a technique related to a disposable diaper in which a hydrophilic nonwoven fabric is used as an exterior sheet 4 that constitutes an exterior body 3 of a diaper 1, the hydrophilic nonwoven fabric having hydrophilized fibers which are obtained by means such as making hydrophobic synthetic fibers undergo treatment with a hydrophilizing agent.

### [CITATION LIST]

### [PATENT LITERATURE]

Patent Literature 1: Japanese Patent Application Publication No. 2017-113186

### SUMMARY

### [TECHNICAL PROBLEM]

In a diaper using a hydrophilic nonwoven fabric such as that of Patent Literature 1, the water absorbency is enhanced compared with a diaper in which the hydrophilic nonwoven fabric is not used. Therefore, there is a risk that the hydrophilic nonwoven fabric absorbs moisture from the atmosphere while the manufactured diaper is distributed in the market, causing mold growth in the exterior body or the like of the diaper. For example, if mold has grown in the diaper at the time when the user opens a new packaged piece and takes out the diaper, the diaper cannot be used even before the diaper is started to be used.

The present invention was achieved in light of conventional problems such as that described above and an aspect of the present invention is to suppress mold growth in an absorbent article including a hydrophilic nonwoven fabric, before starting use.

### [SOLUTION TO PROBLEM]

A main aspect of the present invention for achieving the above-described aspect is an absorbent article including: a liquid-absorbent absorbent main body; and an exterior member that is provided on a non-skin side with respect to the absorbent main body, the exterior member having a hydrophilic nonwoven fabric in at least a part of a farthest-on-non-skin-side surface, the hydrophilic nonwoven fabric being arranged in a manner such that a value that is obtained by dividing a difference between a weight of the absorbent article when opening a packaged absorbent article and a weight of the absorbent article in a dried state, by the weight of the absorbent article in the dried state is equal to or less than 0.1.

Features of the present invention other than the above will become clear by reading the description of the present specification with reference to the accompanying drawings.

### [ADVANTAGEOUS EFFECT OF THE INVENTION]

According to the present invention, it is possible to suppress mold growth in an absorbent article including a hydrophilic nonwoven fabric, before starting use.

### BRIEF DESCRIPTION OF DRAWINGS

FIG. 1 is a schematic perspective view of a diaper 1.
FIG. 2 is a plan view of the diaper 1 in an unfolded and stretched state.
FIG. 3 is a schematic cross-sectional view taken along a line A-A in FIG. 2.
FIG. 4A is a plan view of an absorbent main body 10, and FIG. 4B is a schematic cross-sectional view of the absorbent main body 10.
FIGS. 5A to 5C are explanatory diagrams illustrating the basic principle when moisture is absorbed and evaporated in the diaper 1.
FIGS. 6A to 6C are diagrams illustrating how to fold the diaper 1 when packaging the diaper 1.
FIG. 7 is a schematic cross-sectional view illustrating the structure of the diaper 1 in a folded state.
FIGS. 8A and 8B are diagrams illustrating an example of a case where a single piece of the diaper 1 in a folded state is packaged.
FIGS. 9A and 9B are diagrams illustrating an example of a case where a plurality of diapers 1 in a folded state are packaged being placed side by side.
FIGS. 10A and 10B are explanatory diagrams illustrating a method for attaching a waist elastic member 35 to a front waist portion 30 using welding portions 60.
FIG. 11A is a plan view of a diaper 2 in an unfolded and stretched state.
FIG. 11B is a schematic cross-sectional view taken along a line D-D in FIG. 11A.
FIG. 12A is a plan view of a diaper 3 in an unfolded and stretched state.
FIG. 12B is a schematic cross-sectional view taken along a line E-E in FIG. 12A.
FIG. 13A is a plan view of a diaper 4 in an unfolded and stretched state.
FIG. 13B is a schematic cross-sectional view taken along a line F-F in FIG. 13A.

### DESCRIPTION OF EMBODIMENTS

At least following matters will become clear with description of this specification and attached drawings.

An absorbent article including: a liquid-absorbent absorbent main body; and an exterior member that is provided on a non-skin side with respect to the absorbent main body, the exterior member having a hydrophilic nonwoven fabric in at least a part of a farthest-on-non-skin-side surface, the hydrophilic nonwoven fabric being arranged in a manner such that a value that is obtained by dividing a difference between a weight of the absorbent article when opening a packaged absorbent article and a weight of the absorbent article in a dried state, by the weight of the absorbent article in the dried state is equal to or less than 0.1.

According to the above-described absorbent article, even in the case where the exterior member includes the hydrophilic nonwoven fabric, appropriately arranging the hydrophilic nonwoven fabric suppresses the moisture content of the absorbent article before starting use to the same level as that of an absorbent article not including the hydrophilic nonwoven fabric. Accordingly, mold growth and propagation can be suppressed in the absorbent article before starting use.

In such an absorbent article, it is desirable that the absorbent article has a vertical direction and a lateral direction that intersect with each other, that when being packaged, the absorbent article is folded at at least one location in the vertical direction and the lateral direction, and that in the folded state, at least a part of the hydrophilic nonwoven fabric is not exposed outside.

According to the above-described absorbent article, at least a part of the hydrophilic nonwoven fabric is positioned inside of the absorbent article that is in the folded state, and is in a state of not being exposed to the outside. This reduces the area of a portion in which the hydrophilic nonwoven fabric comes into contact with the atmosphere, and in the distribution process of the absorbent article, the hydrophilic nonwoven fabric is prevented from absorbing moisture (humidity) from the atmosphere. Accordingly, mold growth or the like is suppressed in the absorbent article.

In such an absorbent article, it is desirable that an area of a portion in which the hydrophilic nonwoven fabric is exposed outside in a state where the absorbent article is folded is smaller than 50% of an area of a portion in which the hydrophilic nonwoven fabric is exposed outside in a state where the absorbent article is not folded.

According to the above-described absorbent article, compared with the absorbent article in a state before being folded, the area of a region where the hydrophilic nonwoven fabric may be exposed to the outside and absorb humidity (moisture) from the atmosphere becomes half or less. This can make it likely to suppress mold growth.

In such an absorbent article, it is desirable that in a folded state, at least a part of a surface exposed outside is formed of a hydrophobic nonwoven fabric, the hydrophobic nonwoven fabric having lower hydrophilicity than the hydrophilic nonwoven fabric.

According to the above-described absorbent article, the hydrophobic nonwoven fabric has a portion that is exposed to the outside, and this makes moisture (humidity) less likely to enter the inside of the absorbent article in the folded state. That is, moisture is less likely to reach the hydrophilic nonwoven fabric arranged on the inside of the hydrophobic nonwoven fabric. Accordingly, this can make it likely to suppress mold growth or the like in the absorbent article before use.

In such an absorbent article, it is desirable that the absorbent main body includes an absorbent core containing a superabsorbent polymer, and that when opening the packaged absorbent article, an amount of moisture contained per unit weight of the hydrophilic nonwoven fabric is smaller than an amount of moisture contained per unit weight of the absorbent core.

According to the above-described absorbent article, at least the amount of water absorbed by the hydrophilic nonwoven fabric becomes smaller than the amount of water absorbed by the SAP. Therefore, the amount of water absorbed in the absorbent article as a whole is prevented from becoming excessively high, and mold growth is likely to be suppressed.

In such an absorbent article, it is desirable that the absorbent article has a vertical direction and a lateral direction that intersect with each other, and that the absorbent article has a first fold at which a one-side end region located on a one side in the lateral direction is folded back toward another side in the lateral direction, and a second fold at which an other-side end region located on the other side is folded back toward the one side.

According to the above-described absorbent article, the hydrophilic nonwoven fabric is folded in the transverse direction (lateral direction) so that portions of the hydrophilic nonwoven fabric face each other, and thereby the hydrophilic nonwoven fabric is arranged inside in the thickness direction. This reduces the area of a portion of the hydrophilic nonwoven fabric that is exposed to the outside, enabling to make moisture (humidity) less likely to be absorbed from the atmosphere. Further, the width of the absorbent article is reduced in the transverse direction, and this makes the packaged piece compact, enabling to make it easier to distribute in the market.

In such an absorbent article, it is desirable that the absorbent article has the one-side end region that is folded back at the first fold and the other-side end region that is folded back at the second fold overlap each other when viewed in a thickness direction.

According to the above-described absorbent article, the folded-back portions at least partially overlap each other in the thickness direction, and this makes less likely to form a gap in the folded portion and makes moisture less likely to enter the inner space. Therefore, the hydrophilic nonwoven fabric arranged on the folded inside is less likely to absorb moisture, and mold growth or the like can be suppressed.

In such an absorbent article, it is desirable that the absorbent article has a third fold at which a region located on a one side in the vertical direction is folded back toward another side in the vertical direction.

According to the above-described absorbent article, the hydrophilic nonwoven fabric is folded in the longitudinal direction (vertical direction) so that portions of the hydrophilic nonwoven fabric face each other, and thereby the hydrophilic nonwoven fabric is arranged on the inside in the thickness direction. This reduces the area of a portion of the hydrophilic nonwoven fabric that is exposed to the outside, enabling to make moisture (humidity) less likely to be absorbed from the atmosphere. Further, the length of the absorbent article is reduced in the longitudinal direction, and this makes the packaged piece compact, enabling to make it easier to distribute in the market.

In such an absorbent article, it is desirable that the absorbent main body includes an absorbent core containing a superabsorbent polymer, and that the absorbent article in a folded state has a portion where the absorbent core and the hydrophilic nonwoven fabric overlap each other when the absorbent article is viewed in a thickness direction.

According to the above-described absorbent article, by arranging the hydrophilic nonwoven fabric with overlapping the absorbent core, a part of moisture is absorbed by the hydrophilic nonwoven fabric, and it decreases the amount of moisture that reaches the absorbent core. Therefore, it is possible to reduce the amount of moisture absorbed by the absorbent core. Accordingly, it is possible to reduce the amount of moisture absorbed in the absorbent article as a whole, and this can make it likely to suppress mold growth or the like.

In such an absorbent article, it is desirable that the absorbent article in a folded state has a portion where the absorbent core and a plurality of layers of the hydrophilic nonwoven fabric overlap each other when the absorbent article is viewed in the thickness direction.

According to the above-described absorbent article, by increasing the number of layers of the hydrophilic nonwoven fabric arranged overlapping the absorbent core, it decreases the amount of moisture that permeates through the layer of the hydrophilic nonwoven fabric and reaches the absorbent core. This makes it possible to reduce the amount of moisture absorbed by the absorbent core. Accordingly, it is possible to further reduce the amount of moisture absorbed in the absorbent article as a whole, and this can make it likely to suppress mold growth or the like.

Further, a absorbent-article packaged piece including an absorbent article that is packaged with a packaging member, the absorbent article including: a liquid-absorbent absorbent main body; and an exterior member that is provided on a non-skin side with respect to the absorbent main body, the exterior member having a hydrophilic nonwoven fabric in at least a part of a farthest-on-non-skin-side surface, the hydrophilic nonwoven fabric being arranged in a manner such that a value that is obtained by dividing a difference between a weight of the absorbent article when opening the absorbent-article packaged piece and a weight of the absorbent article in a dried state by the weight of the absorbent article in the dried state is equal to or less than 0.1.

According to the above-described absorbent-article packaged piece, even in the case where the hydrophilic nonwoven fabric is provided on the exterior member of the absorbent article, appropriately arranging the hydrophilic nonwoven fabric can suppress the moisture content of the absorbent article before starting use to the same level as that of an absorbent article not including the hydrophilic nonwoven fabric. Accordingly, mold growth and propagation can be suppressed in the absorbent article before starting use.

In such a absorbent-article packaged piece, it is desirable that the packaging member is a leak-proof sheet member formed of a resin.

According to the above-described absorbent-article packaged piece, the absorbent article is packaged by the leak-proof sheet member (film sheet) formed of the resin, and this can make it likely to prevent moisture from permeating through the packaging member and entering the inside of the packaged piece. Accordingly, the hydrophilic nonwoven fabric of the absorbent article is less likely to absorb moisture, and mold growth or the like can be suppressed in the absorbent article before use.

In such a absorbent-article packaged piece, it is desirable that a single piece of the absorbent article in a folded state is packaged with the packaging member.

According to the above-described absorbent-article packaged piece, a single piece of the absorbent article is packaged (individually packaged) with the leak-proof packaging member, and this makes moisture less likely to enter the inside of the packaged piece (individually-packaged piece), and this can make it likely to prevent the hydrophilic nonwoven fabric from absorbing moisture and causing mold growth in the absorbent article. Further, since the absorbent article can be distributed in the market as a single piece, handling becomes easier, and convenience for users can be enhanced.

In such a absorbent-article packaged piece, it is desirable that a plurality of the absorbent articles in a folded state are packaged with the packaging member, being arranged side by side.

According to the above-described absorbent-article packaged piece, the plurality of absorbent article are packaged together with the leak-proof packaging member, and this makes moisture less likely to enter the inside of the packaged piece, and this can make it likely to prevent the hydrophilic nonwoven fabric from absorbing moisture and causing mold growth in the absorbent article. Further, since a plurality of units of absorbent articles can be distributed in the market, a sufficient amount of absorbent articles can be stably supplied to users.

In such a absorbent-article packaged piece, it is desirable that the absorbent article has a vertical direction, that the absorbent article is folded one time in the vertical direction at a fold at which a region located on a one side in the vertical direction is folded back toward another side in the vertical direction, and that the absorbent article is accommodated such that the fold faces vertically upward.

According to the above-described absorbent-article packaged piece, the heavy absorbent core is likely to be positioned vertically on the lower side and the center of gravity is positioned on the lower side, and therefore the folded state of the absorbent article is likely to maintain, suppressing the loss of the shape of the absorbent article accommodated in the packaged piece. Further, since the folded state is maintained, the hydrophilic nonwoven fabric can be prevented from being exposed to the outside.

In such a absorbent-article packaged piece, it is desirable that in the absorbent article in the folded state, a ratio of an area occupied by the hydrophilic nonwoven fabric to a surface area of a portion located above a vertical center is larger than a ratio of an area occupied by the hydrophilic nonwoven fabric to a surface area of a portion located below the vertical center.

According to the above-described absorbent-article packaged piece, since the ratio of the hydrophilic nonwoven fabric in the lower region is small, the amount of moisture absorbed by the hydrophilic nonwoven fabric can be reduced even in the case where moisture having a heavy specific gravity moves downward. Accordingly, this can make it likely to suppress mold growth or the like.

In such a absorbent-article packaged piece, it is desirable that the absorbent-article packaged piece has an opening guide portion in which a plurality of cuts that penetrate the packaging member in a thickness direction are intermittently arranged side by side along a predetermined direction, and that the absorbent article has a portion where the hydrophilic nonwoven fabric of the absorbent article and the opening guide portion are not in contact with each other.

According to the above-described absorbent-article packaged piece, even when moisture in the atmosphere passes through the cuts of the opening guide portion and enters the inside of the packaged piece, the moisture is less likely to come into contact with the hydrophilic nonwoven fabric. Therefore, this can make it likely to prevent the hydrophilic nonwoven fabric from absorbing the moisture.

In such a absorbent-article packaged piece, it is desirable that the hydrophilic nonwoven fabric of the absorbent article and the entire opening guide portion are not in contact with each other.

According to the above-described absorbent-article packaged piece, even when moisture in the atmosphere passes through the cuts of the opening guide portion and enters the inside of the packaged piece, the moisture is further less likely to come into contact with the hydrophilic nonwoven fabric. Therefore, this can make it likely to prevent the hydrophilic nonwoven fabric from absorbing the moisture.

In such a absorbent-article packaged piece, it is desirable that the absorbent article has a vertical direction, that the absorbent article is folded one time in the vertical direction at a fold at which a region located on a one side in the vertical direction is folded back toward another side in the vertical direction, and that the absorbent article is accommodated such that neither a front surface nor a back surface of the absorbent article that has been folded one time faces the opening guide portion.

According to the above-described absorbent-article packaged piece, a wide area portion of the absorbent article in the folded state does not face the opening guide portion, and this decreases the possibility that the hydrophilic nonwoven fabric comes into contact with moisture that has passed through the cuts of the opening guide portion and entered the inside of the packaged piece. This can make it more likely to suppress the absorption of moisture by the hydrophilic nonwoven fabric.

### First Embodiment

The following describes an absorbent article according to the present invention by way of example of a disposable diaper (hereinafter also referred to as a "diaper 1"). However, the absorbent article according to the present invention includes napkins, panty liners, and other types of absorbent article.

### Configuration of Diaper 1

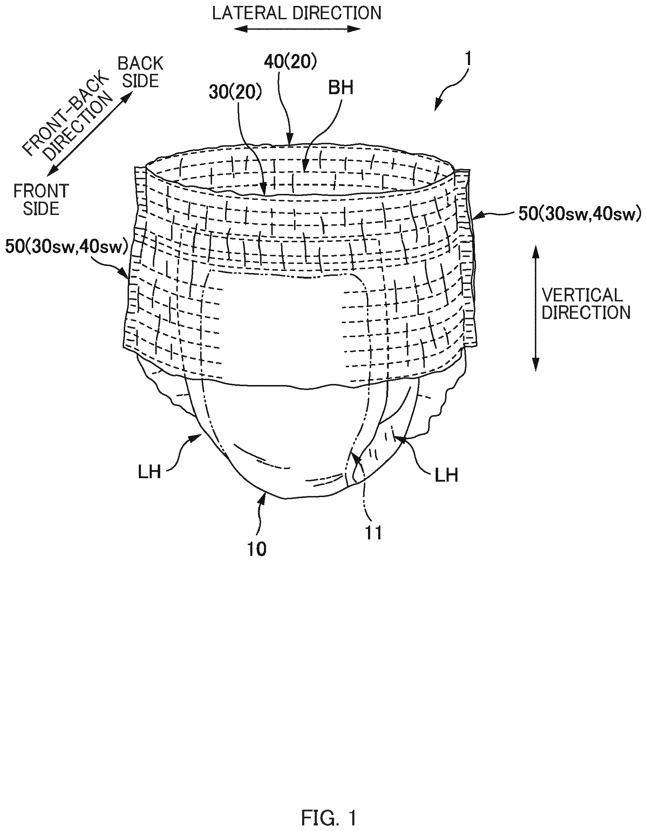

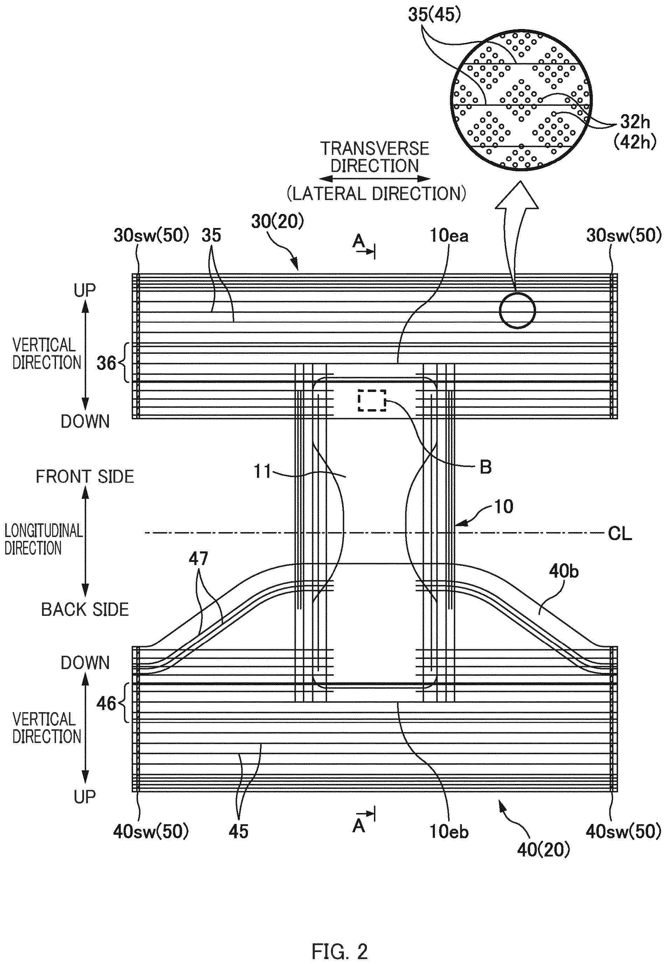

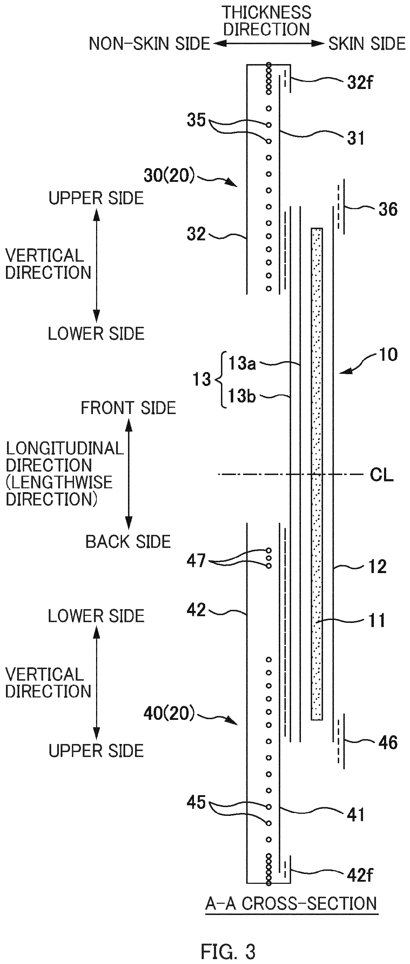

FIG. 1 is a schematic perspective view of the diaper 1. FIG. 2 is a plan view of the diaper 1 in an unfolded and stretched state. FIG. 3 is a schematic cross-sectional view taken along a line A-A in FIG. 2. It should be noted that the "stretched state" of the diaper 1 refers to a state where the entire diaper 1 (the entire product) is stretched without being wrinkled, specifically, a state where the diaper is stretched such that the dimensions of constituent members of the diaper 1 (e.g., an absorbent main body 10, a waist member 20, or the like to be described later) match or are close to the dimensions of the members on their own.

The diaper 1 is a disposable diaper having an underpants shape in a natural state, and in the underpants-shaped state in FIG. 1, the diaper 1 has a vertical direction, a lateral direction, and a front-back direction that intersect with each other and has a waist opening BH and a pair of leg openings LH and LH. The upper side in the vertical direction corresponds to the waist opening BH side, and the lower side corresponds to the crotch side. In addition, the front side in the front-back direction corresponds to the wearer's front side, and the back side corresponds to the wearer's back side. Further, in an unfolded state in FIG. 2, the diaper 1 has a longitudinal direction and a transverse direction that intersect with each other. The longitudinal direction is a direction extending along the vertical direction in FIG. 1 and corresponds to the lengthwise direction of the absorbent main body 10. The transverse direction is a direction extending along the lateral direction in FIG. 1. In addition, as shown in FIG. 3, a direction in which constituent members of the diaper 1 are overlaid is referred to as a thickness direction. In the thickness direction, the side that comes into contact with the wearer's skin is defined as the skin side, and the side opposite to the skin side is defined as the non-skin side.

The diaper 1 has a liquid-absorbent absorbent main body 10 that absorbs excrement, and a waist member 20 that is arranged on the non-skin side of the absorbent main body 10. The waist member 20 is an exterior member that constitutes the exterior of the diaper 1, and has a front waist portion 30 corresponding to the front panel of the diaper 1 and a back waist portion 40 corresponding to the back panel of the diaper 1. That is, the diaper 1 of the first embodiment is a so-called three-piece type underpants-shaped diaper including: as a first component, an absorbent main body 10 that is applied to the wearer's crotch portion and absorbs excrement such as urine or the like; as a second component, a front waist portion 30 that covers the wearer's stomach side portion; and as a third component, a back waist portion 40 that covers the wearer's back side portion.

In an unfolded state in FIG. 2, in a state where the front waist portion 30 and the back waist portion 40 are arranged side by side in the longitudinal direction with a space with respect to each other, lengthwise (longitudinal) end portions 10ea and 10eb of the absorbent main body 10 are respectively joined and fixed to the skin side of the nearest waist portions 30 and 40 while the absorbent main body 10 is spanned between the front waist portion 30 and the back waist portion 40. The external shape thereof forms a substantially H shape in a plan view. Then, from this state, the absorbent main body 10 is folded one time at a fold position, which is the longitudinal central position CL. In this folded state, the front waist portion 30 and the back waist portion 40 that face each other are joined and connected to each other at two lateral side portions 30sw and 40sw to form a pair of side joining portions 50 and 50. That is, the front waist portion 30 and the back waist portion 40 are shaped into an annular shape by the pair of side joining portions 50 and 50. It should be noted that the side joining portion 50 is formed by commonly-known joining means such as welding or adhesive. Accordingly, the diaper 1 is in an underpants-shaped state in which the waist opening BH and the pair of leg openings LH and LH are formed as shown in FIG. 1. Absorbent Main body 10

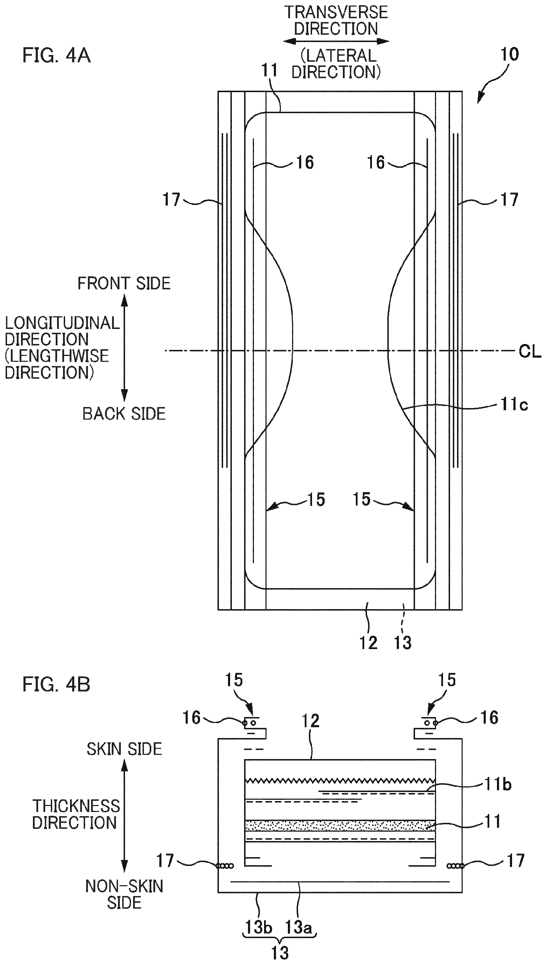

FIG. 4A is a plan view of the absorbent main body 10, and FIG. 4B is a schematic cross-sectional view of the absorbent main body 10.

The absorbent main body 10 has an absorbent core 11 that absorbs excreted fluids, a top sheet 12 that is arranged on the skin side in the thickness direction with respect to the absorbent core 11, and a back sheet 13 that is arranged on the non-skin side with respect to the absorbent core 11. However, the absorbent main body 10 may include other sheet members. For example, a second sheet (not shown) may be provided between the top sheet 12 and the absorbent core 11 in the thickness direction.

The absorbent core 11 is a member that absorbs and holds excreted fluid such as urine, and is formed of, for example, a liquid absorbent fiber (e.g., a pulp fiber) containing a superabsorbent polymer (SAP). It should be noted that the outer circumferential surface of the absorbent core 11 may be covered with a liquid-permeable sheet member (core-wrapping sheet 11b) such as tissue paper or nonwoven fabric. The absorbent core 11 of the present embodiment has a narrow portion 11c, which has a narrow width in the lateral direction, between a front end and a back end in the lengthwise direction, and has a substantially hourglass shape in a plan view as shown in FIG. 4A. This narrow portion 11c is a portion sandwiched between the wearer's two legs while the diaper 1 is put on, and the lateral length thereof is small (the width is narrow), making the absorbent core 11 easier to fit to the wearer's crotch.

The top sheet 12 is a liquid-permeable sheet, and, for example, a hydrophilic air-through nonwoven fabric, spunbond nonwoven fabric, or the like may be used. In the present embodiment, as shown in FIG. 4B, two lateral side portions are folded back toward the non-skin side so as to enclose the absorbent core 11.

The back sheet 13 has a two-layer structure including a liquid-impermeable sheet 13a and an exterior sheet 13b arranged on the non-skin side of the liquid-impermeable sheet 13a. As the liquid-impermeable sheet 13a, a liquid-impermeable and moisture-permeable sheet member may be used. For example, it is possible to use a microporous breathable resin film in which a plurality of fine pores are provided in a sheet mainly made of a resin such as polyethylene or polypropylene. In the present specification, the liquid-impermeable sheet 13a will also be referred to as a "breathable film". That is, the breathable film is a sheet member having "leak-proof property" that does not allow liquids to permeate, but having "moisture permeability" or "breathability" that allows water vapor and air to permeate. On the other hand, as the exterior sheet 13b, a hydrophobic nonwoven fabric having flexibility may be used. For example, it is possible to use an air-through nonwoven fabric, a spunbond nonwoven fabric, or the like.

On two lateral side portions of the absorbent main body 10, a pair of the leak-proof wall portions 15 are respectively provided along the longitudinal direction (the lengthwise direction of the absorbent main body 10). In the present embodiment, the leak-proof wall portion 15 is formed by the above-described exterior sheet 13b. Specifically, in the lateral direction (transverse direction), a part of the exterior sheet 13b is folded toward the skin side at a plurality of locations as shown in FIG. 4B, while extending outward with respect to two end portions of the absorbent core 11, to form the pair of leak-proof wall portions 15. To the skin-side end portion (leading end portion) of each leak-proof wall portion 15, leak-proof-wall elastic members 16 such as elastic strings are attached in a state of being stretched along the longitudinal direction (the lengthwise direction of the absorbent main body 10). While the diaper 1 is put on, the leak-proof wall portions 15 rise toward the wearer's skin side and fit to the wearer's crotch portion due to the stretchability developed by the leak-proof-wall elastic members 16.

Further, to two lateral side portions of the absorbent main body 10, leg elastic members 17 such as elastic strings are attached in a state of being stretched along the longitudinal direction (the lengthwise direction of the absorbent main body 10). While the diaper 1 is put on, the two side portions of the absorbent main body 10 contract and becomes more likely to fit around the wearer's legs due to the stretchability developed by the leg elastic members 17.

### Front Waist Portion 30

As shown in FIG. 3, the front waist portion 30 includes: a skin-side sheet 31 that is arranged farthest on the skin side in the thickness direction; a non-skin-side sheet 32 that is overlaid so as to be adjacent to the non-skin side of the skin-side sheet 31; and waist elastic members 35 that are provided between the skin-side sheet 31 and the non-skin-side sheet 32 in the thickness direction. The front waist portion 30 serving as an exterior member of the diaper 1 basically has a two-layer structure constituted by the skin-side sheet 31 and the non-skin-side sheet 32, but may partially have a configuration of three or more layers including a skin surface sheet 36 or the like described below.

The skin-side sheet 31 and the non-skin-side sheet 32 are sheet members having a rectangular shape in a plan view as shown in FIG. 2, and are formed of, for example, an SMS nonwoven fabric sheet or the like. In the diaper 1, the sheet member (nonwoven fabric sheet) that constitutes the non-skin-side sheet 32 has higher hydrophilicity than the sheet member (nonwoven fabric sheet) that constitutes the skin-side sheet 31. That is, the skin-side sheet 31 is formed of a nonwoven fabric sheet having a low hydrophilicity, and the non-skin-side sheet 32 is formed of a nonwoven fabric sheet having higher hydrophilicity than the skin-side sheet 31. Hereinafter, the nonwoven fabric that constitutes the skin-side sheet 31 will also be referred to as "hydrophobic nonwoven fabric", and the nonwoven fabric that constitutes the non-skin-side sheet 32 will also be referred to as "hydrophilic nonwoven fabric". The "hydrophilicity" of the nonwoven fabric will be described later.

Further, on the surface of the non-skin-side sheet 32, the plurality of hole portions 32h as shown in a partially enlarged view of FIG. 2 are provided. The hole portions 32h are each a through hole that penetrates the non-skin-side sheet 32 in the thickness direction. Providing the hole portions 32h makes it possible to enhance the breathability of the front waist portion 30. Further, arranging the hole portions 32h visibly on the non-skin surface side of the front waist portion 30 makes the user more likely to invoke that the front waist portion 30 has good breathability. Each of the hole portions 32h may have, for example, a circular shape having a diameter of approximately 1 mm, but the shape and arrangement (number and pattern) of the hole portions 32h can be appropriately changed. It should be noted that, in the diaper 1, the through hole corresponding to the hole portion 32h is not provided in the skin-side sheet 31.

The front waist portion 30 of the present embodiment has a folded-back portion 32f in which the upper end portion (the front end portion in the longitudinal direction) of the non-skin-side sheet 32 is folded back from the non-skin side to the skin side and from the front side to the back side in the longitudinal direction. By covering a part (upper end portion) of the skin-side sheet 31 with the folded-back portion 32f, the upper end edge of the skin-side sheet 31 is prevented from biting into the wearer's skin. However, the folded-back portion 32f need not have to be necessarily provided.

Between the skin-side sheet 31 and the non-skin-side sheet 32, a plurality of waist elastic members 35 are arranged side by side in the vertical direction, and are attached in a state of being stretched in the lateral direction. The front waist portion 30 fits around the wearer's front waist due to the stretchability developed by the waist elastic member 35.

The waist elastic members 35 can be attached using an adhesive such as a hot-melt adhesive. For example, it is possible to attach the waist elastic members 35 by applying a hot-melt adhesive to each of them, stretching the waist elastic member 35 at a predetermined stretch factor, and sandwiching the waist elastic member 35 between the skin-side sheet 31 and the non-skin-side sheet 32. That is, the skin-side sheet 31 and the non-skin-side sheet 32 are joined using the adhesive with the waist elastic members 35 interposed therebetween. Further, the waist elastic members 35 may be attached by applying an adhesive to the skin-side sheet 31 side and the non-skin-side sheet 32 side, or the waist elastic member 35 may be attached by welding means in which a later-described welding portion 60 is used.

Further, the front waist portion 30 may have the skin surface sheet 36. As shown in FIG. 3, the skin surface sheet 36 is a sheet member arranged so as to cover the upper end portion (the front end portion in the longitudinal direction) of the absorbent main body 10 from the skin side, and functions as a cover sheet. Accordingly, the upper end edge of the absorbent main body 10 is prevented from biting into the wearer's skin while the diaper 1 is put on. The skin surface sheet 36 is formed of, for example, an SMS nonwoven fabric sheet or the like. It should be noted that the skin surface sheet 36 need not be necessarily provided.

In the front waist portion 30 of the present embodiment, in the case where a sheet member is provided on the skin side with respect to the skin-side sheet 31 or on the non-skin side with respect to the non-skin-side sheet 32, these sheets are arranged so as to cover only a part of the skin-side sheet 31 and the non-skin-side sheet 32. For example, the skin surface sheet 36 in FIG. 3 is provided so as to cover only a part of the skin-side sheet 31, and at least a part of the skin-side sheet 31 is in a state of being exposed to the wearer's skin side.

### Back Waist Portion 40

The back waist portion 40 has substantially the same configuration as the front waist portion 30. That is, the back waist portion 40 includes: a skin-side sheet 41 that is arranged farthest on the skin side in the thickness direction; a non-skin-side sheet 42 that is overlaid so as to be adjacent to the non-skin side of the skin-side sheet 41; and waist elastic members 45 that is provided between the skin-side sheet 41 and the non-skin-side sheet 42 in the thickness direction. Further, similar to the front waist portion 30, the back waist portion 40 may have hole portions 42h, a folded-back portion 42f, a skin surface sheet 46, and the like (see FIGS. 2 and 3). The configuration of the members is substantially the same as that of the front waist portion 30, and therefore description thereof is omitted.

On the other hand, the external shape of the back waist portion 40 is different from the external shape of the front waist portion 30. Specifically, as shown in FIG. 2, the back waist portion 40 has a buttocks cover 40b whose lower portion in the vertical direction with respect to the side joining portion 50 (side portion 40sw) has a substantially trapezoidal shape. The buttocks cover 40b is a portion whose lateral width is narrowed from the upper side toward the lower side in the vertical direction and whose outer edge is curved. By providing the buttocks cover 40b, the back waist portion 40 can widely cover the wearer's buttocks while the diaper 1 is put on.

Further, in the buttocks cover 40b, curved elastic members 47 such as elastic strings as shown in FIG. 2 are provided. The curved elastic members 47 are each attached between the skin-side sheet 41 and the non-skin-side sheet 42 in a state of being stretched along the end edge portion of the buttocks cover 40b. The stretchability developed by the curved elastic members 47 makes the buttocks cover 40b of the back waist portion 40 likely to fit to the wearer's buttocks while the diaper 1 is put on, and also makes it difficult to be turned up from the buttocks.

### Hydrophilicity of Sheet Member

Here, the hydrophilicity of the sheet member will be described. In the diaper 1, a hydrophobic nonwoven fabric is used as the skin-side sheets 31 and 41 that constitute the waist member 20 (the front waist portion 30 and the back waist portion 40) that is the exterior member, and the exterior sheet 13b of the absorbent main body 10. On the other hand, as the non-skin-side sheets 32 and 42 that constitute the waist member 20, a hydrophilic nonwoven fabric having higher hydrophilicity than a hydrophobic nonwoven fabric is used.

The hydrophilic nonwoven fabric of the present embodiment has increased hydrophilicity by undergoing a treatment for attaching a predetermined oil agent to the hydrophobic nonwoven fabric (hydrophilic treatment). As the oil agent used in the hydrophilic treatment, it is possible to use commercially available oil agents having an effect as antistatic agents for fibers, such as anionic oil agents, nonionic oil agents, and blends thereof. These oil agents are put into an oil tank and then subjected to oiling with an oiling roller or the like. As a result, the hydrophilicity of the hydrophobic nonwoven fabric can be enhanced, obtaining the hydrophilic nonwoven fabric. However, the hydrophilic nonwoven fabric may be formed by other methods. For example, a hydrophilic nonwoven fabric may be obtained by manufacturing a nonwoven fabric using highly hydrophilic fibers.

It should be noted that, in the present embodiment, it is assumed that the entire nonwoven fabric that constitutes the non-skin-side sheets 32 and 42 is subject to the hydrophilic treatment, and the hydrophilicity of the entirety of each constituent sheet member is enhanced. However, a configuration is possible in which the hydrophilicity is enhanced only in a partial region of each sheet member. For example, the sheet member may have a locally high hydrophilic portion and a locally low hydrophilic portion by undergoing a hydrophilic treatment only on the partial region of the non-skin-side sheet 32.

The hydrophilicity of a sheet member can be evaluated by measuring a contact angle when ion exchange water is brought into contact with the surface of the sheet member. Specifically, in the case where the contact angle between the hydrophilic nonwoven fabric and the ion exchange water is smaller than the contact angle between the hydrophobic nonwoven fabric and the ion exchange water, the hydrophilicity of the hydrophilic nonwoven fabric becomes higher than the hydrophilicity of the hydrophobic nonwoven fabric. For the hydrophilic nonwoven fabric (non-skin-side sheet 32 or the like) used in the present embodiment, the contact angle with the ion exchange water is preferably less than 90°, and more preferably 50° or less. On the other hand, for the hydrophobic nonwoven fabric (skin-side sheet 31), the contact angle with the ion exchange water is preferably 90° or more, and more preferably 120° or more.

The contact angle can be measured by the following method using, for example, a contact angle meter MCA-J manufactured by Kyowa Interface Science Co., Ltd. First, ion exchange water is dropped (approximately 20 picoliters) onto the surface of the fibers that constitute a sheet member (sheet to be measured), and then immediately the contact angle is measured using the contact angle meter. The measurement is performed at a plurality of places (e.g., five or more places) on the surface of the sheet to be measured, and the average value of these positions is defined as the contact angle. It should be noted that the measurement environment temperature is set to 22°C.

Alternatively, the contact angle may be measured by capturing images of the sheet to be measured onto which ion exchange water is dropped from the cross-sectional direction of the sheet to be measured, analyzing the captured image, and measuring the angle between the ion exchange water droplet and the sheet to be measured.

### Moisture Absorption and Evaporation by Diaper 1

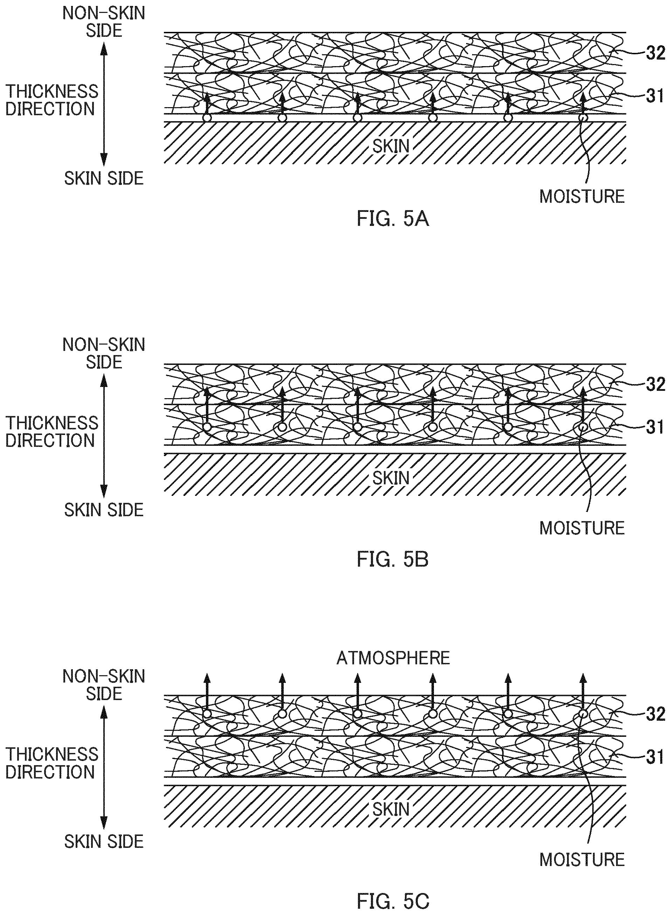

In the diaper 1, the hydrophilic nonwoven fabric having a high hydrophilicity is provided on the non-skin-side surface of the exterior member (the front waist portion 30 and the back waist portion 40), and therefore the diaper 1 can absorb moisture such as sweat from the wearer's skin and make the absorbed moisture to evaporate to the atmosphere. FIGS. 5A to 5C are explanatory diagrams illustrating the basic principle when moisture is absorbed and evaporated in the diaper 1. In FIG. 5, the cross-sections of the front waist portion 30 among the members that constitute the diaper 1 are schematically shown.

First, when the wearer puts on the diaper 1, the skin-side sheet 31 (hydrophobic nonwoven fabric) arranged on the skin side of the front waist portion 30 in the thickness direction comes into contact with the wearer's skin, as shown in FIG. 5A. As described above, the skin-side sheet 31 of the present embodiment is formed of SMS nonwoven fabric and includes meltblown fibers having a small fiber diameter compared with spunbond fibers. Therefore, the thin meltblown fibers have a portion in which the inter-fiber distance becomes narrow by being densely entangled. According to the above-described configuration, a capillary phenomenon occurs in the skin-side sheet 31, and moisture such as sweat attached to the wearer's skin is easily absorbed.

Next, as shown in FIG. 5B, the moisture absorbed by the skin-side sheet 31 is transferred to the non-skin-side sheet 32 (hydrophilic nonwoven fabric) that is overlaid to be adjacent to the non-skin side of the skin-side sheet 31. This is because, while the skin-side sheet 31 is formed of a hydrophobic nonwoven fabric sheet, the non-skin-side sheet 32 is formed of a hydrophilic nonwoven fabric sheet, a difference in the magnitude of hydrophilicity (hydrophilic gradient) is thus generated between the two sheets, and this makes moisture more likely to move from the low hydrophilic skin-side sheet 31 side to the highly hydrophilic non-skin-side sheet 32 side. Therefore, moisture is less likely to be held on the skin-side sheet 31 side that is in contact with the wearer's skin, and is more likely to be held on the non-skin-side sheet 32 side that is not in contact with the wearer's skin.

The moisture held in the non-skin-side sheet 32 is evaporated into the atmosphere from the non-skin-side surface of the non-skin-side sheet 32. In the diaper 1, the non-skin-side sheet 32 is arranged on the outermost (non-skin-side) surface of the absorbent main body 10. That is, the non-skin-side surface of the non-skin-side sheet 32 serves as an interface with the atmosphere. Therefore, the moisture contained in the non-skin-side sheet 32 can be efficiently evaporated to the outside of the diaper 1 from a wide range of the non-skin-side surface. However, it is not necessary that the hydrophilic nonwoven fabric is arranged on the entire farthest-non-skin-side surface of the exterior member. For example, another member different from the non-skin-side sheet 32 (e.g., a tape member) may be provided on a part of the farthest-non-skin-side surface of the exterior member.

As described above, in the diaper 1, by providing the hydrophilic nonwoven fabric (non-skin-side sheets 32 and 42) in the exterior member (waist member 20), it is possible for the diaper 1 to absorb and evaporate the sweat and the like of the wearer while the diaper 1 is put on. However, since there is a possibility that the hydrophilic nonwoven fabric may absorb moisture (humidity) from the atmosphere as well, there is a risk that the exterior member becomes wet in the diaper 1 before use, causing mold propagation. For example, in the case where moisture is absorbed by the hydrophilic nonwoven fabric while the diaper 1 is distributed in the market in a packaged state, there is a risk that, although the diaper 1 is new, the diaper 1 cannot be used because mold has grown when the user opens the package.

Therefore, in the present embodiment, by adjusting the folding method and the packaging method for distribution of the diaper 1 in the market, the hydrophilic nonwoven fabric is prevented from absorbing moisture before use and mold growth is less likely to occur. Hereinafter, means for suppressing the absorption of moisture by the hydrophilic nonwoven fabric will be specifically described.

### Packaging of Diaper 1

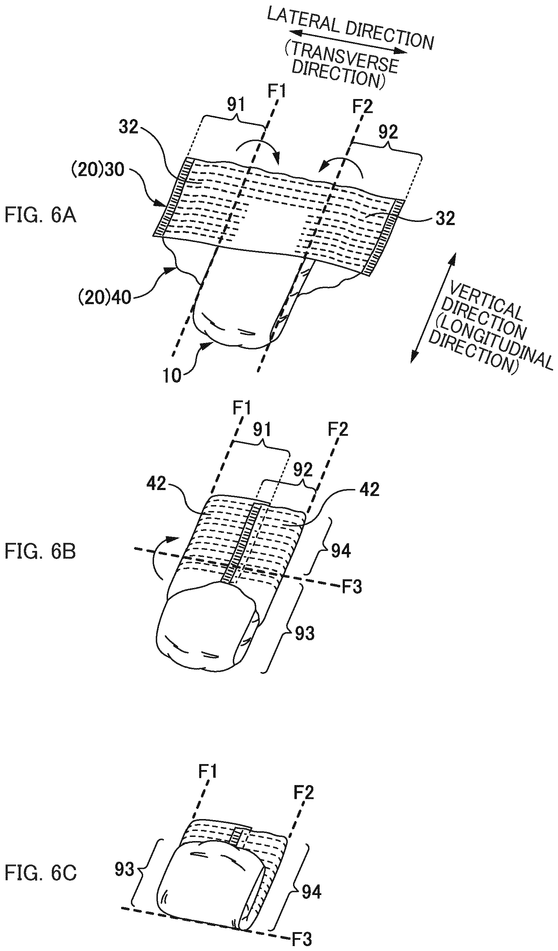

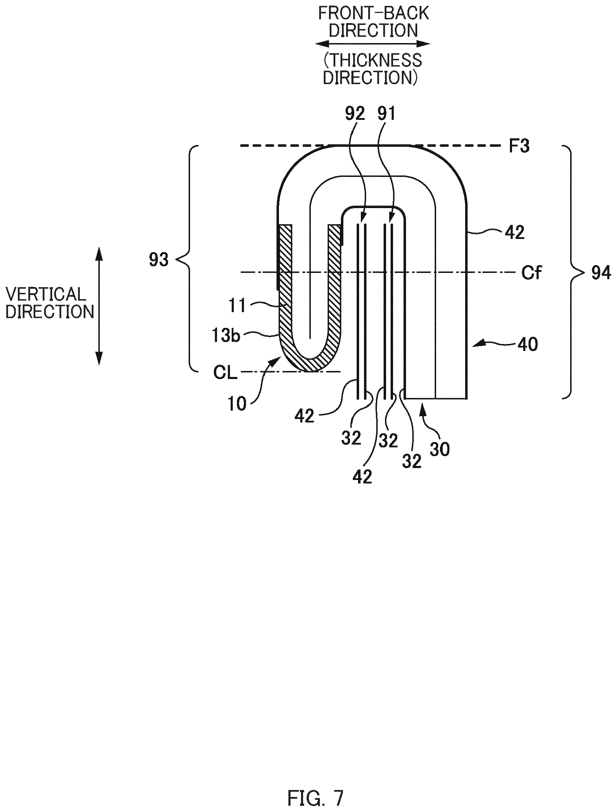

The diaper 1 is distributed on the market in a state of being folded into a compact shape and then wrapped in a predetermined packaging member. FIGS. 6A to 6C are diagrams illustrating how to fold the diaper 1 when packaging the diaper 1. It should be noted that, in FIGS. 6A to 6C, there has been described a method for folding the diaper 1 which is in an underpants-shaped state as in FIG. 1. That is, at the time of starting folding in FIG. 6A, the diaper 1 is folded one time in advance from the unfolded state in FIG. 2, at a longitudinal central position CL. Further, FIG. 7 is a schematic cross-sectional view illustrating the structure of the diaper 1 in the folded state. In FIG. 7, for convenience of description, a part of the configuration (a first part 91, a second part 92, and the like described below) is simply displayed.

As shown in FIG. 6A, first, two end portions of the diaper 1 in the lateral direction (transverse direction) are folded from the outside to the inside. Specifically, an end region 91 of the diaper 1 on the one side in the lateral direction (left side in FIG. 6A) is folded toward the other side in the lateral direction (rightward) at a fold line F1 that extends along the vertical direction (longitudinal direction). Hereinafter, the fold line F1 will also be referred to as a "first fold F1", and the end region 91 of the diaper 1 located on the one side in the lateral direction with respect to the first fold F1 will also be referred to as a "first part 91". Similarly, an end region 92 of the diaper 1 on the other side in the lateral direction (right side in FIG. 6A) is folded toward the one side in the lateral direction (leftward) at a fold line F2 that extends along the vertical direction (the longitudinal direction). Hereinafter, the fold line F2 will also be referred to as a "second fold F2", and the end region 92 of the diaper 1 located on the other side in the lateral direction with respect to the second fold F2 will also be referred to as a "second part 92".

In FIG. 6A, the first fold F1 and the second fold F2 are arranged at positions along two lateral ends of the absorbent main body 10. In the diaper 1, stiffness is different between a portion in which the absorbent main body 10 is arranged and a portion in which the absorbent main body 10 is not arranged, and therefore providing the fold lines F1 and F2 at such positions makes it possible to facilitate the folding of the first part 91 and the second part 92 due to the stiffness difference. However, the arrangement of the first fold F1 and the second fold F2 is not limited thereto. Alternatively, the first fold F1 and the second fold F2 may be arranged at other positions, or the first fold F1 and the second fold F2 may be provided obliquely with respect to the longitudinal direction.

Further, in FIG. 6A, the first part 91 and the second part 92 are folded on the front side of the diaper 1. That is, the front waist portion 30 is folded so as to be located inside and the back waist portion 40 is folded so as to be located outside. However, the front waist portion 30 and the back waist portion 40 may be folded in the opposite direction.

In a state where the first part 91 and the second part 92 are folded, the first part 91 and the second part 92 have portions that overlap each other as shown in FIG. 6B. In other words, the diaper 1 is folded so that the first part 91 and the second part 92 at least partially overlap each other when viewed in the thickness direction. The reason for this will be described later.

Next, from the state in FIG. 6B, the diaper 1 is folded one time in the vertical direction (longitudinal direction) in a manner such that the first part 91 and the second part 92 are located inside. Specifically, a portion 93 of the diaper 1 located on the one side in the vertical direction (lower side in FIG. 6A) is folded at a fold line F3 that extends along the lateral direction (transverse direction) toward the other side in the vertical direction (upward) and is folded so as to overlap the first part 91 and the second part 92. Hereinafter, the fold line F3 will also be referred to as a "third fold F3", the portion of the diaper 1 located on the one side in the vertical direction (lower side) with respect to the third fold F3 will also be referred to as a "third part 93", and the portion located on the other side in the vertical direction (upper side) with respect to the third fold F3 will also be referred to as a "fourth part 94".

It is desirable that the third fold F3 is arranged at a position that does not overlap the absorbent core 11 in the thickness direction. In the diaper 1, the absorbent core 11 has high stiffness, and therefore when folding the diaper 1 at a position that overlaps the absorbent core 11, the folded state is less likely to be maintained due to the restoring force of the absorbent core 11. In the present embodiment, arranging the third fold F3 above the upper end of the absorbent core 11 in the vertical direction (longitudinal direction) makes it easier to maintain the folded state of the diaper 1 (see FIG. 7).

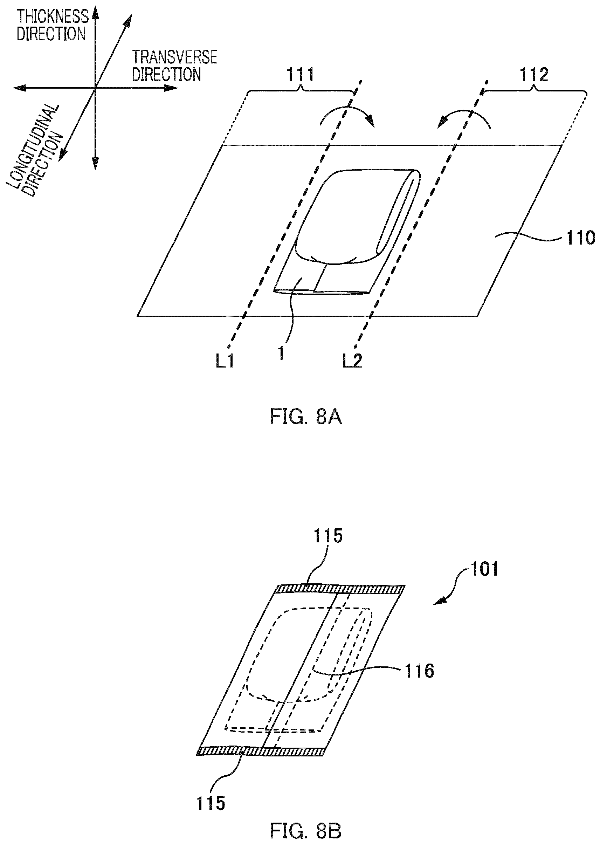

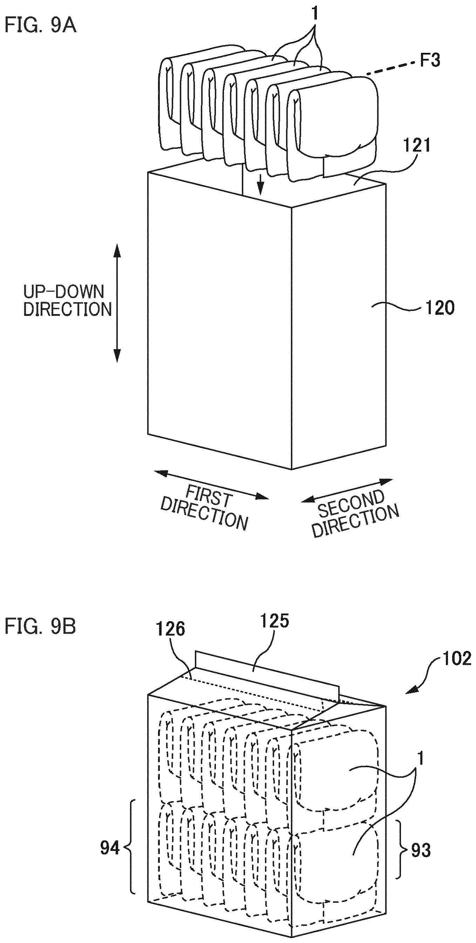

Thus, by folding the diaper 1 at the first fold F1 to the third fold F3, the diaper 1 can be folded in a compact shape as shown in FIG. 6C. In the folded state, the diaper 1 is wrapped by a predetermined packaging member, and the diaper 1 is distributed on the market as a packaged piece of the diaper 1 (see FIGS. 8B and 9B described below).

The diaper 1 in the folded state is folded one time at the vertical (longitudinal) central position CL of the diaper 1, and further folded one time at the third fold F3. That is, the diaper 1 is folded in four from the unfolded state in FIG. 2 (see FIG. 7). At this time, parts of the non-skin-side sheets 32 and 42, which are formed of the hydrophilic nonwoven fabric, are positioned on the inside of the diaper 1 which is in the folded state, and are not in a state being exposed to the outside. For example, in FIG. 7, the non-skin-side sheets 32 and 42 provided in the first part 91 and the second part 92 are sandwiched between the third part 93 and the fourth part 94 in the thickness direction, and are not exposed to the outside (atmosphere). Further, in the fourth part 94, the non-skin-side sheet 32 of the front waist portion 30 is sandwiched between the back waist portion 40 and the third part 93 in the thickness direction, and is not exposed to the outside (atmosphere).

That is, in the diaper 1 in the folded state, the non-skin-side sheets 32 and 42 (hydrophilic nonwoven fabric) of at least the first part 91 and the second part 92 and the non-skin-side sheet 32 (hydrophilic nonwoven fabric) of the fourth part 94 are arranged so as not to be exposed to the outside, and are less likely to come into contact with the atmosphere. Further, by wrapping the diaper 1 in the folded state with a packaging member such as a resin film, it is possible to make the hydrophilic nonwoven fabric less likely to come into contact with the atmosphere. This can make it likely to suppress mold growth caused by the hydrophilic nonwoven fabric becoming wet because of naturally absorbing moisture (humidity) in the atmosphere in the distribution process of the diaper 1.

In the diaper 1 of the present embodiment, the hydrophilic nonwoven fabric is arranged so as not to come into contact with the atmosphere as described above, and this makes it possible to suppress mold growth. Specifically, the hydrophilic nonwoven fabric is arranged in a manner such that the moisture content of the diaper 1 (the packaged piece of the diaper 1) when the diaper 1 in the packaged state is opened is equal to or less than 10%. Here, the "moisture content" refers to a ratio of an increase in the weight of the diaper 1, which becomes heavy due to moisture being contained at the time of opening, to the weight of the diaper 1 which is in a state where moisture is not contained. That is, the hydrophilic nonwoven fabric is arranged in a manner such that a value which is obtained by dividing a difference between the weight of the diaper 1 when the packaged diaper 1 (the packaged piece of the diaper 1) is opened and the weight of the diaper 1 in a dry state, by the weight of the diaper 1 in the dry state, is equal to or less than 0.1.

As described above, the absorbent core 11 of the absorbent main body 10 is configured to include a superabsorbent polymer (SAP) that easily absorbs moisture, and therefore even in the case where the absorbent core 11 does not include a hydrophilic nonwoven fabric, there is a possibility that the absorbent core 11 may absorb a certain amount of moisture and the weight may increase while the absorbent main body 10 is distributed in the market. For example, in a conventional disposable diaper in which the exterior member (corresponding to the waist member 20) is formed only of hydrophobic nonwoven fabric, the SAP absorbs moisture and thereby the diaper may have a certain moisture content at the time of opening from the packaged state.

In contrast, in the diaper 1 of the present embodiment, though the hydrophilic nonwoven fabric (non-skin-side sheets 32 and 42) is provided on the exterior member (waist member 20), the appropriate arrangement of the hydrophilic nonwoven fabric suppresses the absorption of moisture (moisture) before starting use, and keeps the moisture content to be equal to or less than 10%. That is, the moisture content is prevented from becoming excessively high compared with the disposable diaper that does not include a hydrophilic nonwoven fabric, and this makes it possible to suppress mold growth and propagation in the hydrophilic nonwoven fabric.

It should be noted that the "moisture content" can be measured by actually measuring the weight of the diaper 1. For example, an electronic balance or the like is prepared in advance, and the weight of the diaper 1 immediately after the packaged piece is opened is measured and recorded. Next, the diaper 1 is put in an oven and heated at 110°C for 120 minutes to evaporate moisture, and the weight of the diaper in a dry state is measured and recorded. The moisture content at the time of opening of the diaper 1 can be calculated by dividing a difference between the weight of the diaper 1 at the time of opening (the weight containing moisture) and the weight of the diaper 1 in the dry state (the weight not containing moisture) by the weight of the diaper 1 in the dry state. It should be noted that, in the case where a plurality of diapers 1 are accommodated in one packaged piece, at least two or more diapers 1 are weighed by the above-described method, and the arithmetic average value thereof is used as the measurement result.

Note that it is desirable that, at the time of opening of the packaged piece of the diaper 1, the amount of moisture contained per unit weight (g) of the hydrophilic nonwoven fabric is smaller than the amount of moisture contained per unit weight (g) of the absorbent core 11. The superabsorbent polymer (SAP) contained in the absorbent core 11 has a high water absorbency compared with the hydrophilic nonwoven fabric, and easily absorbs moisture. That is, normally, the amount of moisture contained in the absorbent core 11 should be larger than the amount of moisture contained in the hydrophilic nonwoven fabric. In the case where the amount of moisture contained in the hydrophilic nonwoven fabric is larger than the amount of moisture contained in the absorbent core 11, there is a high possibility that a trouble such as intensive moisture contact with the hydrophilic nonwoven fabric may have occurred in the distribution process of the diaper 1. In such a case, the amount of water absorbed in the diaper 1 as a whole becomes excessively high, and there is a risk of mold growth in the hydrophilic nonwoven fabric or the loss of an appropriate water absorption function. In contrast, in the case where, at the time of opening, the amount of moisture contained per unit weight of the hydrophilic nonwoven fabric is smaller than the amount of moisture contained per unit weight of the absorbent core 11, there is a low possibility that an excessive amount of moisture may have been absorbed by the hydrophilic nonwoven fabric at least in the distribution process. Therefore, the possibility that excessive moisture is absorbed in the diaper 1 as a whole becomes low, and mold growth is likely to be suppressed.

Further, as described in FIGS. 6 and 7, when the diaper 1 is folded at least at any point (fold) in the longitudinal direction and in the transverse direction, at least a part of the non-skin-side sheets 32 and 42 (hydrophilic nonwoven fabrics) that constitute the exterior member (waist member 20) is in a state of being not exposed to the outside. Further, it is desirable that the area of a portion in which the non-skin-side sheets 32 and 42 (hydrophilic nonwoven fabric) are exposed to the outside in the folded state is less than 50% of the area of a portion in which the non-skin-side sheets 32 and 42 (hydrophilic nonwoven fabric) are exposed to the outside in a state where the diaper 1 is not folded (underpants-shaped state shown in FIG. 1).

In the present embodiment, as shown in FIG. 6A, the non-skin-side sheet 32 (front waist portion 30) is folded so as to be located inside in the first part 91 and the second part 92, and therefore most of the non-skin-side sheet 32 is not exposed to the outside. Further, as shown in FIG. 6B, the non-skin-side sheet 42 (back waist portion 40) is folded so as to be located inside in the fourth part 94, and therefore a part of the non-skin-side sheet 42 is not exposed to the outside. Accordingly, the diaper 1 is folded in manner such that 50% or more of the entire area of the hydrophilic nonwoven fabric (non-skin-side sheets 32 and 42) provided in the exterior member (waist member 20) of the diaper 1 is not exposed to the outside. Therefore, in the diaper 1 in the folded state, the area of portions of the hydrophilic nonwoven fabric which may absorb humidity (moisture) from the atmosphere is equal to or smaller than half compared with the underpants-shaped state before folding, enabling to be more likely to suppress mold growth.

Further, in the folded state of the diaper 1, at least a part of the surface exposed to the outside is formed of a hydrophobic nonwoven fabric. For example, in FIG. 7, the exterior sheet 13b (hydrophobic nonwoven fabric) of the absorbent main body 10 is exposed on the outer surface of the third part 93. As described in FIG. 5, the hydrophobic nonwoven fabric sometimes absorbs liquid such as sweat due to a capillary phenomenon. However, the hydrophobic nonwoven fabric is less likely to absorb moisture from the atmosphere as a gas (humidity). Therefore, when the hydrophobic nonwoven fabric has a portion that is exposed to the outside, moisture (moisture) is less likely to enter the inside of the hydrophobic nonwoven fabric. Therefore, moisture is less likely to reach the hydrophilic nonwoven fabric or the SAP arranged on the inside of the hydrophobic nonwoven fabric. Accordingly, this can make it likely to suppress mold growth caused by the hydrophilic nonwoven fabric or the like absorbing moisture before the diaper 1 is used.

Further, the diaper 1 in the folded state has the first fold F1 at which the first part 91 located on the one side in the transverse direction (lateral direction) is folded back toward the other side and the second fold F2 at which the second part 92 located on the other side is folded back toward the one side. Accordingly, portions are formed which are folded in the transverse direction (lateral direction) so that portions of the hydrophilic nonwoven fabric face each other. That is, the portions of the hydrophilic nonwoven fabric that face each other are arranged inside in the thickness direction, and therefore the area of the portion that is exposed to the outside becomes small, enabling to make moisture (humidity) less likely to be absorbed from the atmosphere. Further, the diaper 1 is folded so as to have a small transverse width, and this makes the packaged piece of the diaper 1 compact when packaging the diaper 1 with the packaging member, enabling to make it easier to distribute.

At this time, it is desirable that, the first part 91 folded at the first fold F1 and the second part 92 folded at the second fold F2 at least partially overlap each other when viewed in the thickness direction. In the present embodiment, as shown in FIG. 6B, the transverse inner end portion of the folded first part 91 and the transverse inner end portion of the folded second part 92 have portions that overlap each other in the thickness direction. In the case where the first part 91 and the second part 92 do not overlap each other, a gap is likely to generate in the thickness direction at the lateral inner ends of the first part 91 and the second part 92, and there is a risk that moisture (humidity) is more likely to enter the folded inner space from such a gap. In contrast, in the diaper 1, the lateral inner ends of the first part 91 and the second part 92 overlap each other (see FIG. 6B), making it less likely to form a gap at the inner end and making moisture less likely to enter the folded inner space. Therefore, the hydrophilic nonwoven fabric arranged on the folded inside is less likely to absorb moisture, and mold growth can be suppressed.

Further, the diaper 1 in the folded state has the third fold F3 at which the third part 93 located on the one side in the longitudinal direction (vertical direction) is folded back toward the other side. Accordingly, portions are formed which are folded in the longitudinal direction (vertical direction) so that portions of the hydrophilic nonwoven fabric face each other. That is, the portions of the hydrophilic nonwoven fabric that face each other are arranged inside in the thickness direction, and therefore the area of the portion that is exposed to the outside becomes small, enabling to make moisture (humidity) less likely to be absorbed from the atmosphere. Further, the diaper 1 is folded so as to have a short longitudinal length, and this makes the packaged piece of the diaper 1 compact when packaging the diaper 1 with the packaging member, enabling to make it easier to distribute.

Here, the packaged piece in which the diaper 1 is packaged with a predetermined packaging member will be described using a specific example. FIGS. 8A and 8B are diagrams illustrating an example of a case where a single piece of the diaper 1 in the folded state is packaged. FIGS. 9A and 9B are diagrams illustrating an example of a case where a plurality of diapers 1 in the folded state are packaged being placed side by side.

In the case where a single piece of the diaper 1 in the folded state is packaged, first, the diaper 1 in the folded state is arranged onto a packaging member 110 as shown in FIG. 8A. In the present embodiment, the packaging member 110 is a leak-proof film sheet formed of resin, and a polyethylene film or the like can be used, for example. By packaging the diaper 1 with the leak-proof film sheet, it can make it more likely to prevent moisture from permeating through the packaging member and entering the inside of the packaged piece. Accordingly, the hydrophilic nonwoven fabric of the diaper 1 is less likely to absorb moisture, and mold growth or the like can be suppressed in the diaper 1 before use.

It should be noted that, considering only the function of packaging the diaper 1, a sheet member such as nonwoven fabric or paper can be used as the packaging member 110. However, there is a risk that these sheet members allow moisture to permeate, promoting the absorption of moisture by the hydrophilic nonwoven fabric. Accordingly, in the present embodiment, it is preferable to apply the above-described leak-proof sheet member.

After the diaper 1 in the folded state is arranged, two side end portions of the packaging member 110 are folded inward at folding lines L1 and L2 positioned two outer side of the diaper 1 in the transverse direction so as to enclose the diaper 1. In FIG. 8A, in the packaging member 110, a region 111 located outside (on the left side) with respect to the folding line L1 in the transverse direction is folded inward (rightward), and a region 112 located outside (on the right side) with respect to the folding line L2 in the transverse direction is folded inward (leftward).

Then, as in FIG. 8B, portions of the packaging member 110 that are overlaid in the thickness direction are joined to each other along the transverse direction at two longitudinal end portions, forming sealing portions 115 and 115. Finally, the region 111 and the region 112 of the packaging member 110 that are overlaid on each other in the thickness direction are joined along the longitudinal direction, forming the sealing portion 116. Accordingly, a packaged piece 101 is formed in which a single piece of the diaper 1 in the folded state is packaged with the packaging member 110. Hereinafter, the packaged piece 101 will also be referred to as an "individually-packaged piece 101".

As in the packaged piece 101, a single piece of the diaper 1 is packaged (individually packaged) with the leak-proof packaging member 110 and sealed, and this makes moisture less likely to enter the inside of the individually-packaged piece 101, and this can make it likely to prevent the hydrophilic nonwoven fabric from absorbing moisture and causing mold growth in the diaper 1. Further, since the diaper 1 can be distributed in the market as a single piece, handling becomes easier, and convenience for users can be enhanced.

Subsequently, in the case where the plurality of diapers 1 in the folded state are packaged together, first, the packaging member 120 having a bag shape as shown in FIG. 9A is prepared.

Similar to the packaging member 110, the packaging member 120 is a leak-proof film sheet formed of resin, which is shaped into a bag shape having an opening portion 121 on the upper side in the up-down direction. Further, in FIG. 9A, the packaging member 120 is formed in a substantially rectangular parallelepiped shape and has a first direction and a second direction that are orthogonal to each other on a horizontal plane.

In such a bag-shaped packaging member 120, a plurality of diapers 1 in a folded state are accommodated being placed side by side, from the opening portion 121 on the vertical upper side. FIG. 9A shows an example in which seven diapers 1 in a folded state are accommodated in a state of being arranged side by side along the first direction. However, the orientation and the number of diapers 1 to be accommodated in the packaging member 120 are not limited to the example in FIG. 9A. Alternatively, a configuration is acceptable in which after the diaper 1 is made into the individually-packaged piece 101 as shown in FIG. 8B, a plurality of individually-packaged pieces 101 be accommodated in the packaging member 120.

After a predetermined number of diapers 1 are accommodated in the packaging member 120, the opening portion 121 of the packaging member 120 is folded into a so-called gusset fold, and the facing surfaces at the upper end portion are joined to each other along the first direction, forming a sealing portion 125. Accordingly, a packaged piece 102 is formed in which the plurality of diapers 1 in the folded state are packaged with the packaging member 120.

As in the packaged piece 102, the plurality of diapers 1 are packaged together with the leak-proof packaging member 120 and sealed, and this makes moisture less likely to enter the inside of the packaged piece 102, and this can make it likely to prevent the hydrophilic nonwoven fabric from absorbing moisture and causing mold growth in the diaper 1. Further, since a plurality of units of diapers 1 can be distributed in the market, a sufficient amount of diapers 1 can be stably supplied to users.

It should be noted that, in order to facilitate the opening operation of the packaged piece 102, it is desirable that an opening guide portion 126 is provided at a predetermined position of the packaging member 120. In FIG. 9B, on the upper surface portion of the packaged piece 102, the opening guide portion 126 (so-called perforations) is provided in which a plurality of cuts that penetrate the packaging member 120 in the thickness direction are intermittently arranged along the first direction. Accordingly, the user easily performs an operation of opening the packaged piece 102 and taking out the diapers 1 one by one. It should be noted that the position where the opening guide portion 126 is provided is not limited to the upper surface portion of the packaged piece 102. For example, the opening guide portion 126 may be provided in the side surface portion of the packaged piece 102, extending along the up-down direction.

On the other hand, providing the opening guide portion 126 (perforations) causes a risk that moisture in the atmosphere passes through the cuts in the opening guide portion 126 and enters the inside of the packaged piece 102. Therefore, it is desirable that in the packaged piece 102, the diaper 1 is accommodated so as to have a portion where the hydrophilic nonwoven fabric of the diaper 1 in the folded state and the opening guide portion 126 are not in contact with each other. In the case of FIG. 9B, a predetermined space in the up-down direction is provided between the opening guide portion 126 and the accommodated diaper 1. In such a case, the opening guide portion 126 and the hydrophilic nonwoven fabric of the diaper 1 are less likely to be in contact with each other in the packaged piece 102, and therefore the moisture that has permeated through the opening guide portion 126 can be likely to be prevented from being absorbed by the hydrophilic nonwoven fabric.

It should be noted that, in order to easily suppress water absorption by the hydrophilic nonwoven fabric, it is more desirable that the entire opening guide portion 126 is not in contact with the hydrophilic nonwoven fabric of the diaper 1 in the packaged piece 102. For example, it is desirable that the entire opening guide portion 126 is not in contact with the hydrophilic nonwoven fabric by increasing the space (distance) between the opening guide portion 126 and the hydrophilic nonwoven fabric or by separately providing a leak-proof sheet (not shown in FIG. 9B) between the opening guide portion 126 and the hydrophilic nonwoven fabric. In such a case, the opening guide portion 126 and the hydrophilic nonwoven fabric of the diaper 1 are further less likely to be in contact with each other in the packaged piece 102, and therefore water absorption by the hydrophilic nonwoven fabric is more likely to be suppressed, and this can make mold growth or the like less likely to occur.

Further, in the case where the plurality of diapers 1 are accommodated in the packaged piece 102, it is desirable that each diaper 1 is accommodated so that a large area portion of the folded diaper 1 does not face the opening guide portion 126. For example, it is desirable that each diaper 1 is accommodated so that the front surface and the back surface of the diaper 1 which is 1 is folded one time at the third fold F3 (the surfaces corresponding to the third part 93 and the fourth part 94 in FIG. 6C) do not face the opening guide portion 126. The hydrophilic nonwoven fabric (non-skin-side sheet 42) is widely arranged on the outer surface of the fourth part 94 of the diaper 1 (see FIG. 7), and therefore if the fourth part 94 faces the opening guide portion 126, there is a risk that the hydrophilic nonwoven fabric of the fourth part 94 is likely to absorb moisture that has permeated through the opening guide portion 126 and has entered the inside. In contrast, in the packaged piece 102 shown in FIG. 9B, the third part 93 and the fourth part 94 which has a large area in the diaper 1 accommodated being in the folded state and the opening guide portion 126 do not face each other, and thus the hydrophilic nonwoven fabric can be prevented from absorbing moisture.

Further, in the case where a plurality of diapers 1 in the folded state are packaged together as in the packaged piece 102, it is desirable that each diaper 1 is accommodated in the packaging member 120 so that the third fold F3 faces upward in the up-down direction as in FIG. 9A. As described in FIG. 7, the third fold F3 is provided so as not to overlap the absorbent core 11 when the diaper 1 is folded. Therefore, in the case where the third fold F3 is arranged in the diaper 1 in the folded state so as to face vertically upward, there is a high possibility that the heavy absorbent core 11 is positioned vertically on the lower side. That is, there is a high possibility that the center of gravity of the diaper 1 is positioned on the lower side. In such a case, compared with the case where the center of gravity is positioned on the upper side, the shape of the diaper 1 is less likely to lose, and the folded state is likely to maintain. Therefore, while the packaged piece 102 is distributed in the market, the loss of the shape or the like of the diaper 1 accommodated therein is suppressed. Further, by maintaining the folded state, the hydrophilic nonwoven fabric can be prevented from being exposed to the outside.

Further, in the case where the diaper 1 is accommodated so that the third fold F3 faces upward in the up-down direction, in the diaper 1 that is in the folded state, the ratio of the area occupied by the hydrophilic nonwoven fabric to the surface area of a portion located above the vertical central position is larger than the ratio of the area occupied by the hydrophilic nonwoven fabric to the surface area of a portion located below the vertical central position. For example, in FIG. 7, in most of the region located above a vertical central position Cf of the diaper 1 in the folded state, the non-skin-side sheet 42 (hydrophilic nonwoven fabric) is exposed to the outside, and the ratio of the area occupied by the hydrophilic nonwoven fabric to the surface area of the region is high. On the other hand, in the region located below the vertical central position Cf of the diaper 1 in the folded state, the non-skin-side sheet 13b of the absorbent main body 10 is exposed to the outside in the third part 93, and thus the ratio of the hydrophilic nonwoven fabric to the surface area is low.

Generally, moisture such as humidity is more likely to move downward in the up-down direction due to its own weight. Therefore, in the diaper 1, in the case where the ratio of the hydrophilic nonwoven fabric arranged on the surface of the region located below in the up-down direction is large, there is a risk that the hydrophilic nonwoven fabric is likely to absorb moisture. In contrast, in the present embodiment, the ratio of the hydrophilic nonwoven fabric arranged on the surface of the region located below in the up-down direction is smaller than the ratio of the hydrophilic nonwoven fabric arranged on the surface of the region located above. Therefore, as the moisture moves downward, the amount of moisture absorbed by the hydrophilic nonwoven fabric becomes small in the diaper 1 as a whole. Accordingly, this can make it likely to suppress mold growth or the like in the diaper 1.

Further, as shown in FIG. 7, there is a portion where the absorbent core 11 and the hydrophilic nonwoven fabric overlap each other when the diaper 1 in the folded state is viewed in the thickness direction. The diaper 1 in the folded state is likely to come into contact with moisture in the atmosphere, in the third part 93 and the fourth part 94 having a large surface area. Therefore, in the case where the absorbent core 11 is exposed on the surfaces of both the third part 93 and the fourth part 94, there is a risk that a large amount of moisture is absorbed by the SAP having a high water absorbency. In contrast, in the diaper 1, since the hydrophilic nonwoven fabric (the non-skin-side sheets 32 and 42) is arranged on at least the fourth part 94 side with respect to the absorbent core 11 (see FIG. 7), it is possible to reduce the amount of moisture absorbed in the diaper 1 as a whole compared with the case where the absorbent core 11 is arranged in both the third part 93 and the fourth part 94. That is, by arranging the hydrophilic nonwoven fabric with overlapping the absorbent core 11, it is possible to reduce moisture that reaches the absorbent core 11 and to reduce the amount of moisture absorbed by the absorbent core 11. Therefore, with the configuration in which there is a portion where the absorbent core 11 and the hydrophilic nonwoven fabric overlap each other in the thickness direction, it is possible to reduce the amount of moisture absorbed in the diaper 1 as a whole, and mold growth or the like can be likely to be suppressed.

Further, it is preferable that a plurality of layers of the hydrophilic nonwoven fabric are provided in a portion where the absorbent core 11 and the hydrophilic nonwoven fabric overlap in the thickness direction. By increasing the number of layers of the hydrophilic nonwoven fabric arranged overlapping the absorbent core 11, it decreases the amount of moisture that permeates through the layer of the hydrophilic nonwoven fabric and reaches the absorbent core 11, and thus the amount of water absorbed by the absorbent core 11 is further reduced. In the case of FIG. 7, at least six layers of hydrophilic nonwoven fabric (non-skin-side sheets 32 and 42) are provided on the one side (fourth part 94 side) in the thickness direction with respect to the absorbent core 11. Therefore, even when moisture comes into contact with the surface of the fourth part 94, there is a low possibility that moisture permeates through the six layers of hydrophilic nonwoven fabric and reaches the absorbent core 11. Therefore, the amount of moisture absorbed by the absorbent core 11 can be further reduced.

### Modified Example of Method for Attaching Waist Elastic Members 35 and 45

In the above-described embodiment, the waist elastic members 35 and 45 are attached to the waist member 20 by adhering means using a hot-melt adhesive or the like, but the method for attaching the waist elastic members 35 and 45 is not limited thereto. For example, the waist elastic members 35 and 45 may be attached to the waist member 20 using welding means such as ultrasonic welding. It should be noted that, since ultrasonic welding is a commonly-known technique, the description of ultrasonic welding is omitted in the present specification.

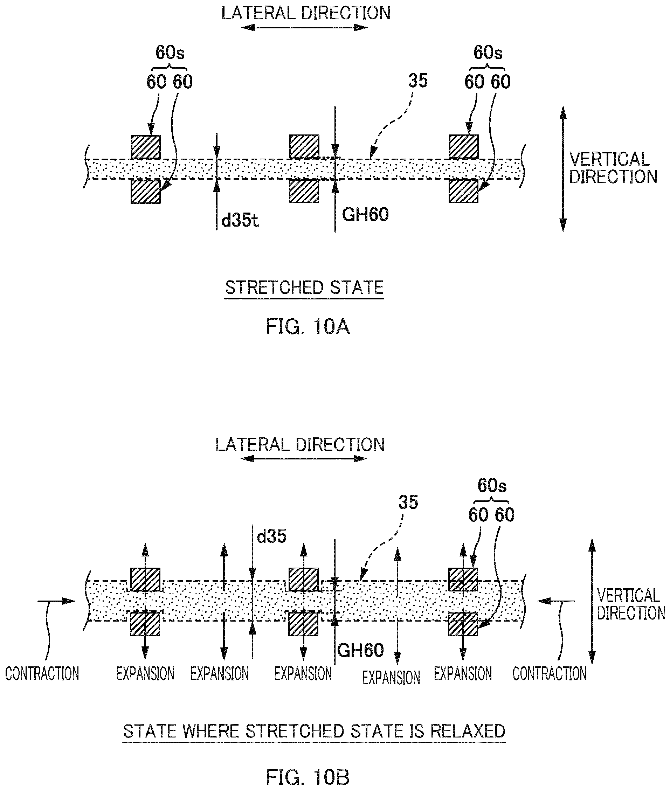

FIGS. 10A and 10B are explanatory diagrams illustrating a method for attaching the waist elastic member 35 to the front waist portion 30 using welding portions 60.

In the present modified example, the waist elastic member 35 is attached to the front waist portion 30 by a plurality of welding portions 60, 60, ..., which are discretely arranged in the lateral direction and the vertical direction. Each of the welding portions 60 is formed into a substantially rectangular shape by ultrasonic welding, and joins the skin-side sheet 31 and the non-skin-side sheet 32 of the front waist portion 30 in the thickness direction. The waist elastic member 35 is attached to the front waist portion 30 by sandwiching the waist elastic member 35 from two vertical sides between welding portion pairs 60s each of which is formed of two welding portions 60 and 60 that are adjacent to each other in the vertical direction.

As shown in FIG. 10A, a pair of welding portions 60 and 60 that constitute the welding portion pair 60s are arranged side by side in the vertical direction with a space GH60. The size of the space GH60 is set to be the same size as or slightly larger than the diameter d35t of the waist elastic member 35 in a state where the waist elastic member 35 is stretched to a predetermined stretch factor (GH60 ≥ d35t). That is, the waist elastic member 35 in the stretched state is arranged between the welding portion pair 60s in the vertical direction.

Next, when the waist elastic member 35 is relaxed from the stretched state, as shown in FIG. 10B, the waist elastic member 35 expands in the vertical direction while contracting in the lateral direction, and the diameter d35 in the natural state becomes larger than the vertical space GH60 of the welding portion pair 60s (d35 > GH60). Accordingly, the waist elastic member 35 is sandwiched between the welding portions 60 and 60 in the vertical direction. As a result, the waist elastic member 35 is attached to the front waist portion 30.

It should be noted that in the diaper 1 in the underpants-shaped state in FIG. 1, the waist elastic members 35 (45) are in a natural state where the waist elastic members 35 (45) are relaxed from the above-described stretched state. Further, in the diaper 1 in the underpants-shaped state, the waist elastic members 35 (45) are joined to the front waist portion 30 (back waist portion 40) by the side joining portions 50 and 50 on two lateral side portions. Therefore, even when the front waist portion 30 (back waist portion 40) is stretched in the lateral direction while the diaper 1 is put on, the waist elastic members 35 (45) are not detached from the waist member 20.

### Second Embodiment

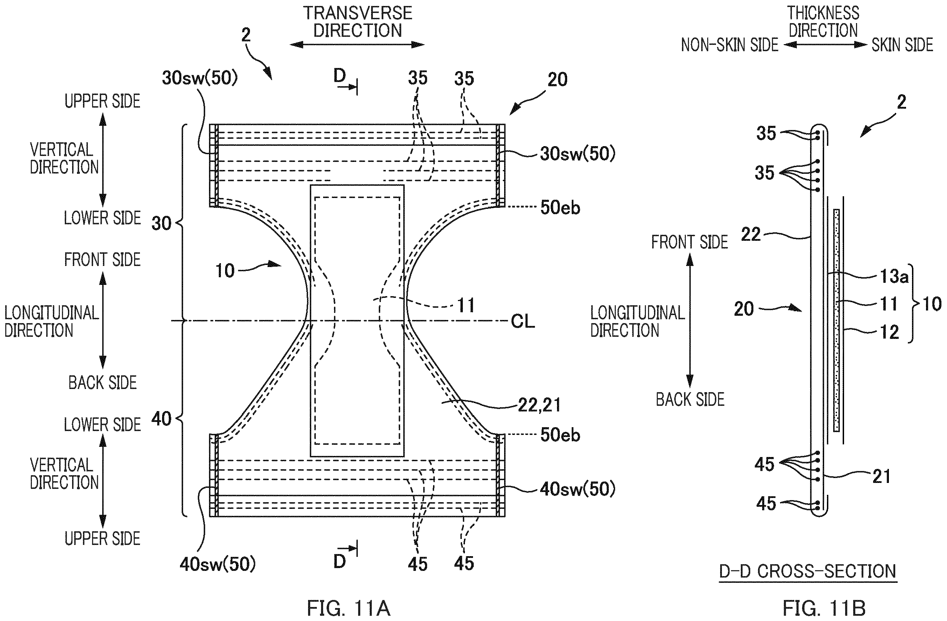

In a second embodiment, an underpants-shaped diaper 2 (hereinafter, also referred to as a "diaper 2") having a configuration partially different from that of the first embodiment will be described. FIG. 11A is a plan view of the diaper 2 in the unfolded and stretched state. FIG. 11B is a schematic cross-sectional view taken along a line D-D in FIG. 11A. It should be noted that the directions (e.g., the longitudinal direction, the transverse direction, or the like) in FIGS. 11A and 11B is the same as the directions defined in the first embodiment.

The diaper 2 of the second embodiment has the liquid-absorbent absorbent main body 10 and the waist member 20 that is joined to the non-skin side of the absorbent main body 10 as an exterior member. Further, as shown in FIGS. 11A and 11B, the waist member 20 of the diaper 2 is integrally constituted so as to be continuous in the longitudinal direction (the lengthwise direction of the absorbent main body 10). That is, the diaper 2 is a so-called two-piece type disposable diaper formed of two parts: the absorbent main body 10 and an exterior member (waist member 20). Hereinafter, in the exterior member (waist member 20) of the diaper 2, a portion located on the front side with respect to the longitudinal central position CL is defined as the front waist portion 30, and a portion located on the back side with respect to the central position CL is defined as the back waist portion 40 (see FIG. 11A).

When shaping the diaper 2 in the unfolded state in FIG. 11A into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, similar to the diaper 1 in FIG. 1, the diaper 2 is in an underpants-shaped state having a waist opening BH and a pair of leg openings LH and LH.

In the diaper 2, the basic configuration and function of the absorbent main body 10 are substantially the same as those of the absorbent main body 10 of the diaper 1 of the first embodiment, and therefore description thereof is omitted. However, the back sheet 13 of the diaper 2 may include only the liquid-impermeable sheet 13a (breathable film) and need not include the exterior sheet 13b. This is because in the diaper 2, the waist member 20 is provided on the entire non-skin-side surface of the absorbent main body 10, and the waist member 20 functions as an exterior sheet of the absorbent main body 10. Therefore, as the back sheet 13 of the absorbent main body 10 of the diaper 2, a sheet member corresponding to the exterior sheet 13b of the diaper 1 need not be provided (see FIG. 11B).

The waist member 20 includes: a skin-side sheet 21; a non-skin-side sheet 22 that is overlaid to be adjacent to the non-skin side of the skin-side sheet 21; and waist elastic members 35 and 45 that are arranged between the skin-side sheet 21 and the non-skin-side sheet 22 in the thickness direction (see FIG. 11B). The skin-side sheet 21 is a hydrophobic nonwoven fabric sheet similar to the skin-side sheets 31 and 41 of the diaper 1. The non-skin-side sheet 22 is a hydrophilic nonwoven fabric sheet similar to the non-skin-side sheets 32 and 42 of the diaper 1. That is, in the diaper 2, the non-skin-side sheet 22 is a nonwoven fabric sheet having higher hydrophilicity than the skin-side sheet 21. Then, similar to the diaper 1, the waist elastic members 35 and 45 are formed of elastic strings or the like, and are attached between the skin-side sheet 21 and the non-skin-side sheet 22 in a state of being stretched in the lateral direction.

Also in the diaper 2 of the second embodiment, the same effect as that of the diaper 1 can be obtained. That is, the hydrophilic nonwoven fabric is arranged in a manner such that when opening the packaged piece of the diaper 2 which is folded and packaged in the same manner as the diaper 1 (see FIGS. 6 to 9), the moisture content of the diaper 2 is equal to or less than 0.1. In other words, the hydrophilic nonwoven fabric is arranged in a manner such that a value which is obtained by dividing a difference between the weight of the diaper 2 at the time of opening the packaged diaper 2 and the weight of the diaper 2 in the dry state by the weight of the diaper 2 in the dry state is equal to or less than 0.1. In such a case, even in the case where the diaper 2 includes a hydrophilic nonwoven fabric, excessive moisture absorption is suppressed, and this makes it possible to suppress mold growth and propagation.

### Third Embodiment

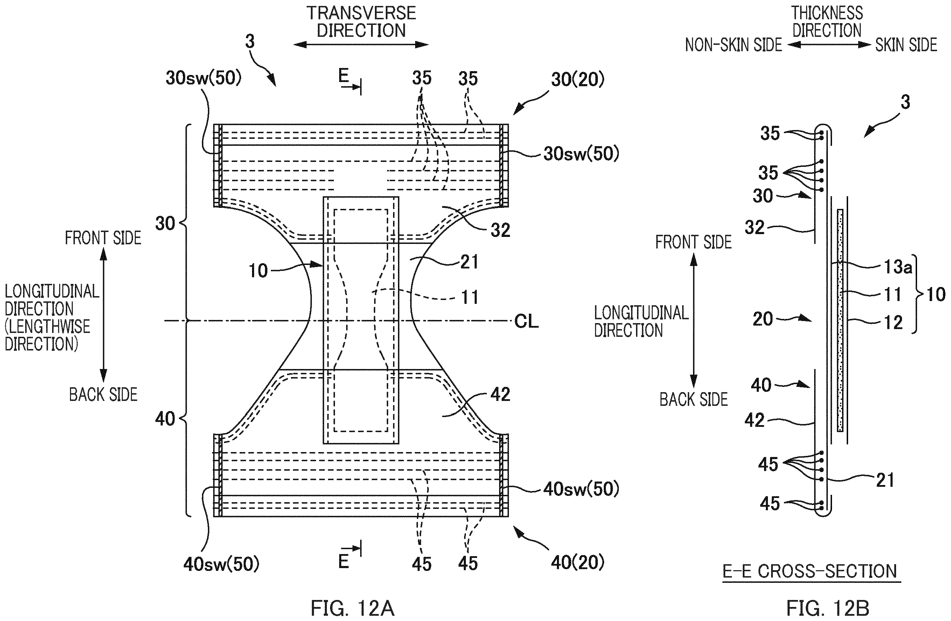

In a third embodiment, an underpants-shaped diaper 3 (hereinafter, also referred to as a "diaper 3") having a configuration partially different from that of the above embodiments will be described. FIG. 12A is a plan view of the diaper 3 in the unfolded and stretched state. FIG. 12B is a schematic cross-sectional view taken along a line E-E in FIG. 12A. It should be noted that the directions (e.g., the longitudinal direction, the transverse direction, or the like) in FIGS. 12A and 12B is the same as the directions in the first embodiment.

The diaper 3 of the third embodiment has the liquid-absorbent absorbent main body 10 and the waist member 20 that is joined to the non-skin side of the absorbent main body 10 as an exterior member. As shown in FIGS. 12A and 12B, the waist member 20 of the diaper 3 is integrally constituted so as to be continuous in the longitudinal direction (the lengthwise direction of the absorbent main body 10). That is, the skin-side sheet 21 is configured as an integral sheet member that is continuous from an end portion located on the one side to an end portion located on the other side in the longitudinal direction. On the other hand, the non-skin-side sheet 32 arranged on the longitudinal front side (front non-skin-side sheet) and the non-skin-side sheet 42 arranged on the longitudinal back side (back non-skin-side sheet) are configured as different sheet members that are non-continuous in the longitudinal direction. A diaper having such a structure will also be referred to as a simple three-piece type disposable diaper. Further, hereinafter, in the exterior member (waist member 20) of the diaper 3, a portion located on the front side with respect to the longitudinal central position CL is defined as the front waist portion 30, and a portion located on the back side with respect to the central position CL is defined to as the back waist portion 40 (see FIG. 12A) .

When shaping the diaper 3 in the unfolded state in FIG. 12A into an underpants shape, the absorbent main body 10 and the waist member 20 are folded one time at a fold position, which is the longitudinal central position CL.

In this folded state, the front waist portion 30 and the back waist portion 40 that face each other are joined and connected to each other at two lateral side portions 30sw and 40sw to form the pair of side joining portions 50 and 50. Accordingly, similar to the diaper 1 in FIG. 1, the diaper 3 is in an underpants-shaped state having a waist opening BH and a pair of leg openings LH and LH.

In the diaper 3, the basic configuration and function of the absorbent main body 10 are substantially the same as those of the absorbent main body 10 of the diaper 2 of the second embodiment, and therefore description thereof is omitted.

In the exterior member (waist member 20) of the diaper 3, the front waist portion 30 includes: the single skin-side sheet 21 that is located on the skin side in the thickness direction and extends from one end side (front side) to the other end side (back side) in the longitudinal direction; the non-skin-side sheet 32 that is located on the front side in the longitudinal direction and is overlaid to be adjacent to the non-skin side of the skin-side sheet 21; and the waist elastic members 35 that are arranged between the skin-side sheet 21 and the non-skin-side sheet 32 in the thickness direction. The back waist portion 40 includes: the skin-side sheet 21 that is common to the front waist portion 30; the non-skin-side sheet 42 that is located on the back side in the longitudinal direction and is overlaid to be adjacent to the non-skin side of the skin-side sheet 21; and the waist elastic members 45 that are arranged between the skin-side sheet 21 and the non-skin-side sheet 42 in the thickness direction.

The skin-side sheet 21 is a hydrophobic nonwoven fabric sheet similar to the skin-side sheets 31 and 41 of the diaper 1. The non-skin-side sheets 32 and 42 are hydrophilic nonwoven fabric sheets similar to the non-skin-side sheets 32 and 42 of the diaper 1. That is, in the diaper 3, the non-skin-side sheets 32 and 42 are nonwoven fabric sheets having higher hydrophilicity than the skin-side sheet 21. Then, similar to the diaper 1, the waist elastic members 35 and 45 are formed of elastic strings or the like, and are attached between the skin-side sheet 21 and the non-skin-side sheets 32 and 42 in a state of being stretched in the lateral direction.

Also in the diaper 3 of the third embodiment, the same effect as that of the diaper 1 can be obtained. That is, the hydrophilic nonwoven fabric is arranged in a manner such that when opening the packaged piece of the diaper 3 which is folded and packaged in the same manner as the diaper 1 (see FIGS. 6 to 9), the moisture content of the diaper 3 is equal to or less than 0.1. In other words, the hydrophilic nonwoven fabric is arranged in a manner such that a value which is obtained by dividing a difference between the weight of the diaper 3 at the time of opening the packaged diaper 3 and the weight of the diaper 3 in the dry state by the weight of the diaper 3 in the dry state is equal to or less than 0.1. In such a case, even in the case where the diaper 3 includes a hydrophilic nonwoven fabric, excessive moisture absorption is suppressed, and this makes it possible to suppress mold growth and propagation.

### Fourth Embodiment

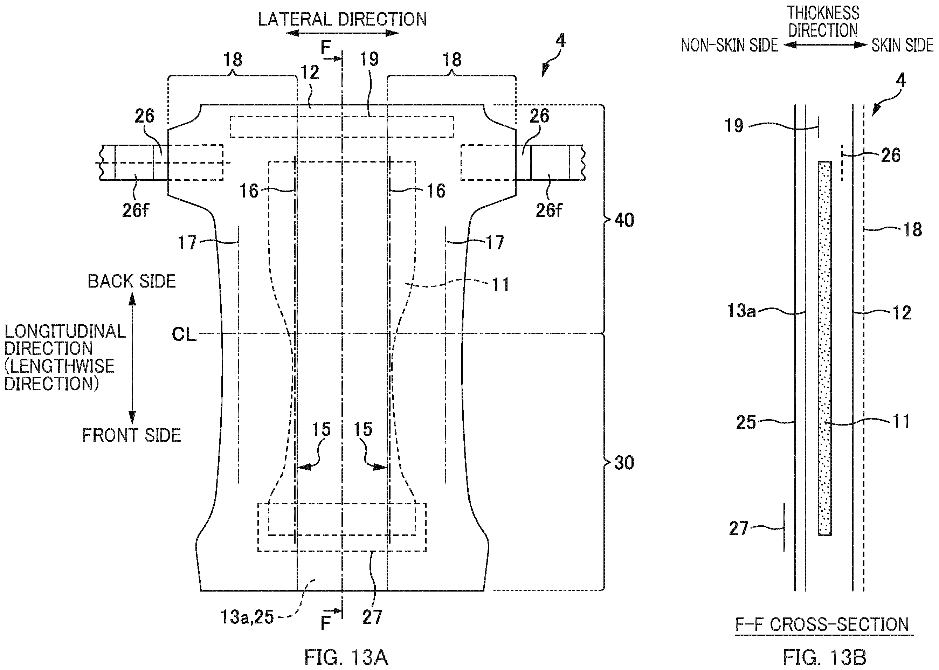

In a fourth embodiment, a "tape-type diaper" different from the "underpants-shaped diaper" described in the first to third embodiments (hereinafter, also referred to as a" diaper 4") will be described as an example of the absorbent article. FIG. 13A is a plan view of the diaper 4 in the unfolded and stretched state. FIG. 13B is a schematic cross-sectional view taken along a line F-F in FIG. 13A. It should be noted that the directions (e.g., the longitudinal direction, the transverse direction, or the like) in FIGS. 13A and 13B is the same as the directions in the first embodiment.

As shown in FIGS. 13A and 13B, the diaper 4 includes: the absorbent core 11 that absorbs excrement; the liquid-permeable top sheet 12 that is located on the skin side with respect to the absorbent core 11; the liquid-impermeable sheet 13a that is located on the non-skin side with respect to the absorbent core 11; an exterior sheet 25 that is located on the non-skin side with respect to the liquid-impermeable sheet 13a; and a pair of side sheets 18 that are joined to the skin side of the top sheet 12 in two lateral side portions of the top sheet 12. Further, the leg elastic members 17 (e.g., elastic strings) that stretch and contract in the longitudinal direction are arranged on two lateral side portions of the diaper 4.

The absorbent core 11, the top sheet 12, and the liquid-impermeable sheet 13a of the diaper 4 are portions corresponding to the absorbent main body 10 in the diaper 1, and respectively have the same functions as the absorbent core 11, the top sheet 12, and the liquid-impermeable sheet 13a that constitute the absorbent main body 10 of the diaper 1. For example, the liquid-impermeable sheet 13a is a breathable film that allows moisture such as urine absorbed by the absorbent core 11 to permeate as water vapor.

The exterior sheet 25 of the diaper 4 is an exterior member corresponding to the waist member 20 in the diaper 1, and is formed of hydrophilic nonwoven fabric, similar to the non-skin-side sheets 32 and 42 that constitute the waist member 20 of the diaper 1. It should be noted that the exterior member of the diaper 4 may have the same configuration as the waist member 20 in the diaper 1. For example, a configuration is also acceptable in which hydrophobic nonwoven fabric (not shown in FIG. 13B) is provided adjacent to the skin side of the exterior sheet 25 formed of hydrophilic nonwoven fabric, and the exterior member of the diaper 4 is formed of two sheet members.

Hereinafter, for convenience of description, a portion located on the front side with respect to the longitudinal central position CL of the diaper 4 is defined as the front waist portion 30, and a portion located on the back side with respect to the central position CL is defined as the back waist portion 40 (see FIG. 13A).

The pair of side sheets 18 are each a liquid-permeable nonwoven fabric sheet similar to the top sheet 12. The lateral inner end portions of the side sheets 18 are provided with the leak-proof-wall elastic members 16 such as elastic strings that can stretch and contract in the longitudinal direction. While the diaper 4 is put on, the lateral inner end portions of the side sheets 18 rise toward the wearer's skin side due to the contractive force developed by the leak-proof-wall elastic members 16, forming the leak-proof wall portions 15 in the same manner as the diaper 1.

On the back side of the diaper 4 in the longitudinal direction (back waist portion 40), a pair of fastening tapes 26 that extend outward on two lateral sides are provided. On the skin-side surfaces located on the free end sides (outside in the lateral direction) of the fastening tapes 26, hook-and-loop fasteners 26f including a plurality of hook members (not shown) are provided. Further, on the non-skin-side surface of the diaper 4 on the front side in the longitudinal direction (front waist portion 30), a target sheet 27 for engaging the fastening tapes 26 when putting on the diaper 4 is provided. The target sheet 27 is a member that can be engaged with the hook-and-loop fasteners 26f (hook members) of the fastening tapes 26, and is formed of, for example, nonwoven fabric.

Further, on the back side of the diaper 4 in the longitudinal direction (back waist portion 40), back elastic members 19 that stretch and contract along the lateral direction (transverse direction) are provided. The back elastic member 19 is a band-shaped elastic member formed of a stretchable nonwoven fabric or the like, and is attached to the diaper 4 in a state of being stretched in the lateral direction. The stretching/contracting force developed by the back elastic member 19 makes the diaper 4 more likely to fit around the wearer's waist at the time of putting the diaper 4 on the wearer's body.

When putting on the diaper 4, for example, the diaper 4 in the unfolded state shown in FIG. 13A is arranged in the wearer's crotch portion, the front waist portion 30 is applied to the wearer's stomach side portion, and the back waist portion 40 is applied to the wearer's back side portion (buttocks). Then, the pair of fastening tapes 26 and 26 are wrapped from the back side to the front side along the wearer's waist, and the hook-and-loop fasteners 26f are engaged with the target sheet 27 on the wearer's stomach side. This makes it possible to form the waist opening BH and the pair of leg openings LH and LH substantially similar to those of the diaper 1, and to fix the position of the diaper 4 to the wearer's body (crotch portion).

It should be noted that the target sheet 27 need not be necessarily provided in the diaper 4. For example, the diaper 4 may be put on by directly engaging the hook-and-loop fasteners 26f with the nonwoven fabric that constitutes the exterior sheet 25, instead of arranging the target sheet 27 on the non-skin-side surface of the exterior sheet 25.

Also in the diaper 4 of the fourth embodiment, the same effect as that of the diaper 1 can be obtained. That is, the hydrophilic nonwoven fabric is arranged in a manner such that when opening the packaged piece of the diaper 4 which is folded and packaged in the same manner as the diaper 1 (see FIGS. 6 to 9), the moisture content of the diaper 4 is equal to or less than 0.1. In other words, the hydrophilic nonwoven fabric is arranged in a manner such that a value which is obtained by dividing a difference between the weight of the diaper 4 at the time of opening the packaged diaper 4 and the weight of the diaper 4 in the dry state by the weight of the diaper 4 in the dry state is equal to or less than 0.1. In such a case, even in the case where the diaper 4 includes a hydrophilic nonwoven fabric, excessive moisture absorption is suppressed, and this makes it possible to suppress mold growth and propagation.

### Other Embodiments

Although the above embodiments of the present invention have been described, but the above-described embodiments are intended to facilitate the understanding of the present invention and are not intended to limit the interpretation of the present invention. In addition, the present invention can be modified or improved within the scope of the gist of the present invention, and it is needless to say that equivalents thereof are included in the present invention.

### REFERENCE SIGNS LIST

1: diaper (absorbent article, underpants-shaped diaper) (first embodiment),
2: diaper (absorbent article, underpants-shaped diaper) (second embodiment),
3: diaper (absorbent article, underpants-shaped diaper) (third embodiment),
4: diaper (absorbent article, tape-type diaper) (fourth embodiment),
10: absorbent main body,
11a: absorbent core, 11b: core-wrapping sheet, 11c: narrow portion,
12: top sheet,
13: back sheet,
13a: liquid-impermeable sheet (breathable film), 13b: exterior sheet,
15: leak-proof wall portion, 16: leak-proof-wall elastic member, 17: leg elastic member,
18: side sheet, 19: back elastic member,
20: waist member,
21: skin-side sheet (hydrophobic nonwoven fabric),
22: non-skin-side sheet (hydrophilic nonwoven fabric),
25: exterior sheet (hydrophilic nonwoven fabric),
26: fastening tape, 26f: hook-and-loop fastener, 27: target sheet,
30: front waist portion, 30sw: side portion,
31: skin-side sheet (hydrophobic nonwoven fabric),
32: non-skin-side sheet (hydrophilic nonwoven fabric), 32f: folded-back portion, 32h: hole portion,
35: waist elastic member,
36: skin surface sheet,
40: back waist portion, 40b: buttocks cover, 40sw: side portion,
41: skin-side sheet (hydrophobic nonwoven fabric),
42: non-skin-side sheet (hydrophilic nonwoven fabric), 42f: folded-back portion, 42h: hole portion,
45: waist elastic member,
46: skin surface sheet, 47: curved elastic member,
50: side joining portion,
60: welding portion,
60s: welding portion pair,
91: first part (end region), 92: second part (end region),
93: third part, 94: fourth part,
101: packaged piece (individually-packaged piece), 102: packaged piece (plurality),
110: packaging member,
111: region, 112: region, 115: sealing portion, 116: sealing portion,
120: packaging member,
121: opening portion, 125: sealing portion, 126: opening guide portion (perforation),
F1: first fold, F2: second fold, F3: third fold,
BH: waist opening, LH: leg opening,
CL: central position (longitudinal direction, lengthwise direction)

## Claims

1. An absorbent article comprising:
a liquid-absorbent absorbent main body; and
an exterior member that is provided on a non-skin side with respect to the absorbent main body,
the exterior member having a hydrophilic nonwoven fabric in at least a part of a farthest-on-non-skin-side surface,
the hydrophilic nonwoven fabric being arranged in a manner such that a value that is obtained by dividing a difference between a weight of the absorbent article when opening a packaged absorbent article and a weight of the absorbent article in a dried state, by the weight of the absorbent article in the dried state is equal to or less than 0.1.

2. The absorbent article according to claim 1, wherein
the absorbent article has a vertical direction and a lateral direction that intersect with each other,
when being packaged, the absorbent article is folded at at least one location in the vertical direction and the lateral direction, and
in the folded state, at least a part of the hydrophilic nonwoven fabric is not exposed outside.

3. The absorbent article according to claim 2, wherein
an area of a portion in which the hydrophilic nonwoven fabric is exposed outside in a state where the absorbent article is folded
is smaller than
50% of an area of a portion in which the hydrophilic nonwoven fabric is exposed outside in a state where the absorbent article is not folded.

4. The absorbent article according to any one of claims 1 to 3, wherein
in a folded state, at least a part of a surface exposed outside is formed of a hydrophobic nonwoven fabric,
the hydrophobic nonwoven fabric having lower hydrophilicity than the hydrophilic nonwoven fabric.

5. The absorbent article according to any one of claims 1 to 4, wherein
the absorbent main body includes an absorbent core containing a superabsorbent polymer, and
when opening the packaged absorbent article,
an amount of moisture contained per unit weight of the hydrophilic nonwoven fabric is smaller than an amount of moisture contained per unit weight of the absorbent core.

6. The absorbent article according to any one of claims 1 to 5, wherein
the absorbent article has a vertical direction and a lateral direction that intersect with each other, and
the absorbent article has
a first fold at which a one-side end region located on a one side in the lateral direction is folded back toward another side in the lateral direction, and
a second fold at which an other-side end region located on the other side is folded back toward the one side.

7. The absorbent article according to claim 6, wherein
the absorbent article has the one-side end region that is folded back at the first fold and the other-side end region that is folded back at the second fold overlap each other when viewed in a thickness direction.

8. The absorbent article according to claim 6 or 7, wherein
the absorbent article has a third fold at which a region located on a one side in the vertical direction is folded back toward another side in the vertical direction.

9. The absorbent article according to claim 8, wherein
the absorbent main body includes an absorbent core containing a superabsorbent polymer, and
the absorbent article in a folded state has a portion where the absorbent core and the hydrophilic nonwoven fabric overlap each other when the absorbent article is viewed in a thickness direction.

10. The absorbent article according to claim 9, wherein
the absorbent article in a folded state has a portion where the absorbent core and a plurality of layers of the hydrophilic nonwoven fabric overlap each other when the absorbent article is viewed in the thickness direction.

11. An absorbent-article packaged piece comprising an absorbent article that is packaged with a packaging member,
the absorbent article including:
a liquid-absorbent absorbent main body; and
an exterior member that is provided on a non-skin side with respect to the absorbent main body,
the exterior member having a hydrophilic nonwoven fabric in at least a part of a farthest-on-non-skin-side surface,
the hydrophilic nonwoven fabric being arranged in a manner such that a value that is obtained by dividing a difference between a weight of the absorbent article when opening the absorbent-article packaged piece and a weight of the absorbent article in a dried state by the weight of the absorbent article in the dried state is equal to or less than 0.1.

12. The absorbent-article packaged piece according to claim 11, wherein
the packaging member is a leak-proof sheet member formed of a resin.

13. The absorbent-article packaged piece according to claim 11 or 12, wherein
a single piece of the absorbent article in a folded state is packaged with the packaging member.

14. The absorbent-article packaged piece according to claim 11 or 12, wherein
a plurality of the absorbent articles in a folded state are packaged with the packaging member, being arranged side by side.

15. The absorbent-article packaged piece according to claim 14, wherein
the absorbent article has a vertical direction,
the absorbent article is folded one time in the vertical direction at a fold at which a region located on a one side in the vertical direction is folded back toward another side in the vertical direction, and
the absorbent article is accommodated such that the fold faces vertically upward.

16. The absorbent-article packaged piece according to claim 15, wherein
in the absorbent article in the folded state,
a ratio of an area occupied by the hydrophilic nonwoven fabric to a surface area of a portion located above a vertical center is larger than a ratio of an area occupied by the hydrophilic nonwoven fabric to a surface area of a portion located below the vertical center.

17. The absorbent-article packaged piece according to any one of claims 14 to 16, wherein
the absorbent-article packaged piece has an opening guide portion in which a plurality of cuts that penetrate the packaging member in a thickness direction are intermittently arranged side by side along a predetermined direction, and
the absorbent article has a portion where the hydrophilic nonwoven fabric of the absorbent article and the opening guide portion are not in contact with each other.

18. The absorbent-article packaged piece according to claim 17, wherein
the hydrophilic nonwoven fabric of the absorbent article and the entire opening guide portion are not in contact with each other.

19. The absorbent-article packaged piece according to claim 17 or 18, wherein
the absorbent article has a vertical direction,
the absorbent article is folded one time in the vertical direction at a fold at which a region located on a one side in the vertical direction is folded back toward another side in the vertical direction, and
the absorbent article is accommodated such that neither a front surface nor a back surface of the absorbent article that has been folded one time faces the opening guide portion.
